# EUROPÄISCHE PATENTSCHRIFT

(11) **EP 1 421 106 B1**
(45) Veröffentlichungstag und Bekanntmachung des Hinweises auf die Patenterteilung: **21.01.2009**
(21) Anmeldenummer: 02796249.7
(22) Anmeldetag: 20.08.2002
(51) Int. Cl.: C07K 5/06, C07K 5/08, C07K 5/10, A61K 38/00

(54) **KATALYSE DER CIS-TRANS ISOMERISIERUNG VON SEKUNDÄREN-AMID PEPTIDVERBINDUNGEN**
CATALYSIS OF THE CIS/TRANS ISOMERISATION OF SECONDARY AMIDE PEPTIDE COMPOUNDS
CATALYSE DE L'ISOMERISATION CIS/TRANS DE COMPOSES SECONDAIRES D'AMIDES ET DE PEPTIDES

(30) Priorität: 20.08.2001 DE 10140777
(43) Veröffentlichungstag der Anmeldung: 26.05.2004
(73) Patentinhaber: Max-Planck-Gesellschaft zur Förderung der Wissenschaften e.V., Berlin (DE)
(72) Erfinder: SCHIENE-FISCHER, Cordelia, 06120 Halle (DE); FISCHER, Gunter, 06120 Halle (DE); HABAZETTL, Judith, Maria, 4125 Riehen (CH); KÜLLERTZ, Gerhard, 06120 Halle (DE)
(74) Vertreter: Vossius & Partner
(86) Internationale Anmeldenummer: PCT/EP2002/009300
(87) Internationale Veröffentlichungsnummer: WO 2003/018618

(56) Entgegenhaltungen:
- WO-A-98/30535
- US-A- 5 266 576
- RUEDIGER S ET AL: "Modulation of substrate specificity of the DnaK chaperone by alteration of a hydrophobic arch" JOURNAL OF MOLECULAR BIOLOGY, LONDON, GB, Bd. 304, Nr. 3, 2000, Seiten 245-251, XP002227984 ISSN: 0022-2836 in der Anmeldung erwähnt
- RUEDIGER S ET AL: "Substrate specificity of the DnaK chaperone determined by screening cellulose-bound peptide libraries" EMBO JOURNAL, OXFORD UNIVERSITY PRESS, SURREY, GB, Bd. 16, Nr. 7, 1. April 1997 (1997-04-01), Seiten 1501-1507, XP002227985 ISSN: 0261-4189 in der Anmeldung erwähnt
- MAYER M P ET AL: "MOLECULAR BASIS FOR INTERACTIONS OF THE DNAK CHAPERONE WITH SUBSTRATES" BIOLOGICAL CHEMISTRY, XX, XX, Bd. 381, Nr. 9/10, September 2000 (2000-09), Seiten 877-885, XP009004374 ISSN: 1431-6730
- "SYNTHESIS, STABILITY, AND BIOLOGICAL EVALUATION OF WATER-SOLUBLE PRODRUGS OF A NW ECHINOCANDIN LIPOPEPTIDE. DISCOVERY OF A POTENTIAL CLINICAL AGENT FOR THE TREATMENT OF SYSTEMIC CANDIDIASIS AND PNEUMOCYSTIC CARINII PNEUMONIO (PCP)" JOURNAL OF MEDICINAL CHEMISTRY, AMERICAN CHEMICAL SOCIETY. WASHINGTON, US, Bd. 35, Nr. 1, 10. Januar 1992 (1992-01-10), Seiten 194-198, XP000571471 ISSN: 0022-2623
- "Catalogue Bachem Peptides and Biochemicals Germany, " 1999 , BACHEM BIOCHEMICA GMBH , HEIDELBERG, DE XP002227986 VERBINDUNGEN G-1000 (AC-ALA-ALA-OH) UND G-3970 (AC-ALA-GLU-OH) Seite 162 -Seite 163
- DATABASE WPI Section Ch, Week 199002 Derwent Publications Ltd., London, GB; Class D21, AN 1990-013076 XP002227989 & JP 01 294800 A (AJINOMOTO KK), 28. November 1989 (1989-11-28)
- HANESSIAN S ET AL: "Tetrahydrofuran as a Scaffold for Peptidomimetics. Application to the Design and Synthesis of Conformationally Constrained Metalloproteinase Inhibitors" TETRAHEDRON, ELSEVIER SCIENCE PUBLISHERS, AMSTERDAM, NL, Bd. 56, Nr. 39, 22. September 2000 (2000-09-22), Seiten 7643-7660, XP004217265 ISSN: 0040-4020
- LI M ET AL: "Structure-based design and synthesis of novel thrombin inhibitors based on phosphinic peptide mimetics" BIOORGANIC & MEDICINAL CHEMISTRY LETTERS, OXFORD, GB, Bd. 9, Nr. 14, 19. Juli 1999 (1999-07-19), Seiten 1957-1962, XP004171618 ISSN: 0960-894X
- SCHIENE-FISCHER CORDELIA ET AL: "The hsp70 chaperone DnaK is a secondary amide peptide bond cis-trans isomerase." NATURE STRUCTURAL BIOLOGY, Bd. 9, Nr. 6, Juni 2002 (2002-06), Seiten 419-424, XP001156574 ISSN: 1072-8368 (ISSN print)
- DATABASE CA [ONLINE] [Online] CHEMICAL ABTRACTS SERVICE, COLUMBUS, OHIO, US; MHASKAR, S.Y. ET AL.: "Synthesis of N-acyl amino acids and correlation of structure with surfactant properties of their sodium salts " retrieved from STN Database accession no. 114:209578 XP002227988 & JOURNAL OF THE AMERICAN OIL CHEMISTS' SOCIETY, Bd. 67, Nr. 12, 1990, Seiten 1015-1019,

## Beschreibung

### Beschreibung der Erfindung

Die vorliegende Erfindung beruht auf dem Befund, daß die cis/trans Isomerisierung sekundärer-Amid Peptidbindungen in Oligo- und Polypeptiden katalytisch beschleunigt werden kann. Diese Katalyse wird durch im Weiteren als "sekundär-Amid Peptidbindung cis-trans Isomerasen" (APIasen) bezeichnete Enzyme bewirkt. Es ist davon auszugehen, daß die APIase Aktivität in einer Vielzahl von pathophysiologischen Prozessen eine zentrale Rolle spielt. Die Erfindung betrifft somit Arzneimittel, umfassend Substanzen, die die APIase Aktivität hemmen.

Es ist bekannt, dass in Oligo- und Polypeptiden die Rotation um die Bindung, die üblicherweise durch den Diederwinkel omega (ω) definiert ist und die sich zwischen dem Carbonyl-C-Atom und dem Stickstoffatom befindet, gegenüber anderen C-N Bindungen z. B. in aliphatischen Dialkylaminen, behindert ist. Die quantenchemische Beschreibung der Ursache liefert ein Bild, daß durch die Ausbildung einer partiellen CN-Doppelbindung beschrieben werden kann (z.B.. L. Stryer, Biochemistry, ISBN 3-89330-690-0). Auch sind im Peptidrückgrat weitere, weniger gehinderte Rotationen um Bindungen möglich, die üblicherweise durch die Winkel psi (ψ) und phi (Φ) beschrieben werden. Die Verhältnisse dieser Winkel in der Polypeptidkette definieren im wesentlichen die Raumstruktur von Peptiden oder Proteinen. Diese Gegebenheiten sind dem Fachmann bekannt und lassen sich derzeit entweder direkt mittels NMR-Spektroskopie oder Röntgenstrukturanalyse messen, bzw. auch mittels Raumkonturdiagrammen, den *Ramachandran-Plots,* (Ramachandran, et al., 1968, Adv. Prot. Chem., 23:283-437)vorhersagen und darstellen.

Die Ausbildung definierter Raumstrukturen von Peptiden oder Proteinen, vom Fachmann als Proteinfaltung (Gething and Sambrook, 1992, Nature 355:33-45) bezeichnet, ist wesentlich für die biologische Funktion von Peptiden oder Proteinen. Die definierte Faltung von Proteinen (Tertiärstruktur) ist wichtig zur Herstellung biologisch aktiver Moleküle und sie geht nach der Verknüpfung der Aminosäurebausteine zur Primärstruktur vor sich. Auch zahlreiche biologische Funktionen basieren auf einer Änderung der räumlichen Struktur von Peptiden oder Proteinen, wobei oft nur Teilbereiche der Polypeptidkette einer Veränderung unterliegen Solche Veränderungen sind für vielfältige biochemische Abläufe beschrieben (Wie-Jia O. et al., 1995, J. Biol. Chem., 270:18051-18059), wie zum Beispiel beim Transport von Proteinen durch Membranen (Quilty JA. and Reithmeier RAF, 2000, Traffic 1:987-998). Von den pathobiochemischen Prozessen, die mit Strukturänderungen von Proteinen einhergehen, zu nennen sind hier Krankheiten wie Cystische Fibrose, Juveniles Lungenemphysem, Tay-Sachs Syndrome, Kongenitale Sucrose Isomaltase Defizienz oder Familiäre Hypercholesterolaemie, ist besonders das bei der spongiformen Encephalopathie auftretenden Scrapie-Prion-Protein (PrP^{Sc}) besonders gut untersucht. Dabei unterscheidet sich die Raumstruktur des PrP^{Sc} stark von der Struktur des Prion Proteins (PrP^{C}) Gesunder, obwohl die Primärstrukturen von PrP^{Sc} und PrP^{C} gleich sind (Prusiner, 1991, Science 252:1515-1522). Die der Bildung falsch gefalteter Proteine zu Grunde liegenden Prozesse sind derzeit nicht immer bekannt. So ist derzeit noch vollkommen unbekannt, wie sich *in vivo* aus PrP^{C} das falsch gefaltete PrP^{Sc} bildet. Auch *in vitro* ist dieser Prozess noch unverstanden.

Demgegenüber ist die Bildung eines nativen Proteins aus seiner ungefalteten Peptidkette mittels biotechnologischer Prozessführung für einzelne Proteine gut beschrieben. Dabei zeigt sich, dass die Faltung der ungeordneten/ungefalteten Peptidkette zum nativen Protein schnelle und langsame Schritte beinhaltet. Einer der bekanntesten langsamen Faltungsschritte wird durch die *cis*/*trans* Isomerisierung der tertiär-Amid Prolyl-Peptidbindungen (R-CO-X-R' mit R,R'= Aminoacyl oder Peptidyl; X= cyclo(-NCH(CONHR')-CH₂CH₂CH₂-) verursacht (z.B.: Eberhardt ES. et al., 1996, JACS 118:12261-12266), deren biologischen und chemischen Eigenschaften sehr unterschiedlich von sekundären-Amid Peptidbindungen (-RC(O)NHR') sind.

Wie durch zahlreiche wissenschaftliche Untersuchungen belegt ist, wird durch Katalyse dieser Isomerisierung mittels Peptidyl-Prolyl *cis*/*trans* Isomerasen (Nomenklatur-Nr. EC 5.2.1.8), wie z.B. mittels FK506-bindender Proteine (FKBP's) (Dumont FJ, 2000, Current Medicinal Chemistry 7:731-48) die Faltungsgeschwindigkeit dieses langsamen Faltungsschrittes *in vitro* als auch *in vivo* beschleunigt (Fischer G, 1994, Angew. Chemie Intl.ed. Engl. 33: 1415-1436), während Vertreter dieser Enzymklasse die *cis*/*trans* Isomerisierung sekundärer-Amid Peptidbindungen in Oligo- und Polypeptiden nicht merklich katalysieren können (Scholz et al., 1998, Biol. Chem. 379, 361-365).

Wie kürzlich gezeigt werden konnte, beinhaltet auch die Faltung prolinfreier Proteine langsame Faltungsschritte (Pappenberger G. et al., 2001, Nature Structural Biology 8:452-458). Die Ursache ist offensichtlich in der temporären Ausbildung von Proteinformen mit sekundären-Amid Peptidbindungen in einer unnatürlichen *cis* Konformation zu sehen, die sich in die biologisch aktive Raumstruktur des nativen Proteins nicht einpassen kann. Gegenüber der *cis*/*trans* Isomerisierung von Prolyl-Peptidbindungen ist die *cis*/*trans* Isomerisierungsgeschwindigkeit von sekundären-Amid Peptidbindungen, die 19 der 20 gencodierten Aminosäuren ausbilden, ca. 100-fach schneller.

Es wurde überraschend gefunden, daß sich die cis/trans Isomerisierung von Peptiden, die sekundäre-Amid Peptidbindungen enthalten, in wäßrigen Medien mittels katalytischen Mengen an Substanzen (Katalysatoren) spezifisch beschleunigen läßt. So wird durch Zugabe eines Homogenates aus Escherichia coli oder eines daraus isolierten Proteins (DnaK) (Beispiel 7) zum Meßansatz die Geschwindigkeit der *cis*/*trans* Isomerisierung einer vielfach größeren Menge z.B. der Alanyl-Tyrosin-Peptidbindung in Ala-Ala-Tyr-Ala-Ala oder z.B. der Ala-Leu Peptidbindung in Alanylleucin spezifisch und in wiederholten Zyklen über einen Zeitraum von Wochen ohne Aktivitätsverlust beschleunigt (katalysiert), ohne daß als Nebenreaktion die Peptidbindung zerstört oder das Oligopeptid chemisch anderweitig verändert wird (Beispiele 1 und 8). Eine entsprechende endogen enzymatische Aktivität wird auch in Säugern vermutet.

Gegenstand der überraschend gefundenen Katalyse sind Peptidbindungen in Oligopeptiden und Proteinen des Typs Xaa-Yaa. Wobei Xaa alle natürlicher Amino- und Iminosäuren einbezieht und Yaa alle natürlichen Aminosäuren beinhaltet, Iminosäuren aber ausschließt. Gegenstand der Katalyse sind auch sekundäre-Amid Peptidbindungen, die aus chemisch veränderten Aminosäuren gebildet werden. Solche Aminosäuren entstehen durch posttranslationale Modifikationen von Oligopeptiden und Proteinen *in vivo* (z.B. Williams KR. Stone KL., 1995, Methods in Molecular Biology 40:157-75). Eine Katalyse der *cis*/*trans* Isomerisierung von sekundären-Amid Peptidbindungen durch die aus biologischen Material isolierten Katalysatoren auf Proteinbasis (Enzyme), im weiteren als "Sekundär-Amid Peptidbindungs *cis*/*trans* Isomerasen" (APIasen) bezeichnet (englisch: Secondary Amide Peptide Bond *cis*/*trans* Isomerases), wird dann beobachtet, wenn sich durch Zugabe einer notwendigen, aber immer katalytischen Menge des Enzyms unter geeigneten Bedingungen eine Beschleunigung der *cis*/*trans*-Isomerisierung der beobachteten sekundären-Amid Peptidbindung nachweisen lässt. Eine Beschleunigung durch APIasen ist dann zu beobachten, wenn die Isomerisierungsgeschwindigkeit größer als die fehlerbehaftete Geschwindigkeit ohne APIase ist. Unter optimalen Bedingungen liegt die notwendige Katalysatormenge unter 0.01% der Konzentration des Moleküls, welches die zu katalysierende Peptidbindung enthält. Es kann aber auch notwendig sein, die notwendige Katalysatormenge um ein Vielfaches höher zu wählen als die Konzentration des Moleküls, welches die zu katalysierende Peptidbindung enthält.

Hierbei werden wie dem Fachmann bekannt unter dem Begriff "Peptid" Kondensationsprodukte aus zwei oder mehr Aminosäuren mit Säureamidartiger Verknüpfung verstanden, unter dem Begriff "Oligopeptid" werden insbesondere Peptide mit zwei bis zehn Aminosäureresten verstanden.

Vorzugsweise wird eine erfindungsgemäße Katalyse in Pufferlösungen, wie z.B. 0.1 m Phosphatpuffer, pH 7.4 beobachtet. Die genutzten wässrigen Medien können aber auch aus Mehrphasensystemen bestehen, wie sie sich z.B. durch Kombinationen von Polymeren mit chaotropen Reagentien bilden, wie dies z.B. in US5723310 beschrieben wird. Die Protonenkonzentration der wässrigen Lösung, in dem die erfindungsgemäße Katalyse abläuft, muß dabei einen Betrag haben, der die katalytische Funktion des Katalysators nicht wesentlich, d.h. um nicht mehr als 98% mindert. Ausführungsformen der Erfindung können den Katalysator in gelöster Form, aber auch an feste Oberflächen gebunden, bzw. auch in Mikrostrukturen kompartimentiert, wie z.B. verkapselt nutzen.

Im Sinne der Erfindung ist die spezifische Katalyse der *cis*/*trans* Isomerisierung von sekundären-Amid Peptidbindungen eine wesentliche Eigenschaft dieser Katalysatoren. Als spezifisch im Sinne der Erfindung wird die zeitlich befristete Ausbildung eines Komplexes zwischen Katalysator und Substrat verstanden, wobei die biochemischen Konstanten dieses Komplexes, wie Bildungs- und Zerfallsgeschwindigkeit in die gewünschte Reaktionsrichtung nicht nur von der Wechselwirkung des Katalysators mit der Peptidbindung selbst, sondern auch von der unmittelbaren Wechselwirkung des Katalysators mit dieser Peptidbindung benachbarten chemischen Funktionalitäten beeinflusst werden können (sogenannte secondary binding-sites). Auch ist z.B. die Katalyse der *cis*/*trans*-Isomerisierung von Peptidbindungen durch Protonen oder Hydroxylionen unspezifisch, da das wesentliche Merkmal einer spezifischen Katalyse, die Unterdrückung von Nebenreaktionen (hier z. B. die Hydrolyse der sekundären-Amid Peptidbindung) und die Beschleunigung nur der gewünschten Reaktion nicht gegeben ist. Ein Beipiel für eine spezifische APIase-Katalyse ist in Beispiel 8 gegeben. Neben der unspezifischen Katalyse der *cis*/*trans* Isomerisierung der sekundären-Amid Peptidbindungen durch Protonen oder Hydroxylionen lässt sich auch die relativ gut untersuchte (Zusammenfassung bei C.Cox und T. Lecta, 2000, Accounts of Chemical Research 33:849-858) Katalyse durch Metall-Ionen (Lewis-Säuren) aufführen. Sie läßt sich von der erfindungsgemäßen Katalyse durch die Spezifität in wässrigen Lösungen unterscheiden, da die verwendeten Lewis-Säuren Nebenreaktionen induzieren (z.B. Peptidbindungshydrolyse: Grant KB, Patthabi S., 2001, Anal. Biochem. 289:196-201) oder Seitenkettenoxidationen (Huang XD et al., 1999, Biochem. 38:7609-16), Li SH. et al., 1995, Biotech&Bioengineering 48:490-500) oder selbst als Reaktionspartner Zou J, Sugimoto N., 2000, Biometals 13:349-359); Casalaro et al., 2001, Polymer 42:903-912); Sun S. et al., 2000, Organic Letters 2:911-914) unter Verbrauch der Ausgangsmaterialien zu unerwünschten Produkten fungieren.

Die Struktur und Konformation des sich zeitlich ausgebildenden Komplexes zwischen APIase und einem Substrat, kann, bei Vorliegen der räumlichen Struktur genutzt werden, um Vorhersagen für Inhibitoren zu treffen. So kann mittels der bekannten räumlichen Peptid-bindenden Struktur der APIase DnaK (Wang H. et al., 1998, Biochemistry, 37:7929-7940, Zhu X. et al., 1996, Science 272:1606-1614) und dem Fachmann bekannten empirischen Berechnungen (z.B.: Kasper P. et al., 2000, Proteins 40:185-192) und Bindungstudien von DnaK zu Peptidbibliotheken (Rüdiger S. et al., 1997, EMBO J. 16:1501-1507; Rüdiger S. et al., 2000, J Mol Biol 304:245-251; Mayer MP. et al., 2000, Biol Chem, 381:877-885) die Bindungstasche des Proteins DnaK vorhergesagt werden als hydrophobe Bindungsseite für drei Aminsäurereste, flankiert von negativ geladenen Resten, die basische Aminosäurereste binden können.

Die überraschende Entdeckung, dass DnaK APIase-Aktivität besitzt, ermöglicht es unter Einbeziehung von APIase-Aktivitätsmessungen und ihrer Inhibierung die aus den Strukturdaten und Bindungsstudien erhaltenen Daten so zu präzisieren, dass diese zum Auffinden von Inhibitoren der APIase-Aktivität von DnaK geeignet, bzw. auch zum Ausschluß von Peptiden als Inhibitoren geeignet sind. Durch das Auffinden der APIase-Aktivität können. Inhibitoren dieser Aktivität gefunden werden. Die vorliegende Erfindung stellt Arzneimittel bereit, die APIase-Aktivitäten von Proteinen inhibieren können.

Die Verbindungen der erfindungsgemäßen Arzneimittel schließen auch solche Inhibitoren von APIasen ein, welche die Struktur und Konformation des APIase-Substrates imitieren, wenn es im aktiven Zentrum der APIase gebunden ist.

Die Verbindungen des erfindungsgemäßen Arzneimittel haben eine typische Inhibierungskonstante von 100. mikromolar oder weniger. Eingeschlossen sind auch organische Moleküle, welche die Struktur und Konformation einer Peptidbindung R2-R3 imitieren die an APIasen binden und deren APIase-Aktivität dadurch inhibieren, wenn R2 für alle natürlichen Aminosäuren steht und R3 folgende Aminosäuren einschiebt: Methionin, Alanin, Serin, Glutaminsäure, Leucin, Lysin, Isoleucin und Glycin.

Die Verbindungen der erfindungsgemäßen Arzneimittel schliessen Verbindungen ein, die aus einer Kernregion (Bindungsmotif) bestehen, welches die Struktur und Konformation einer Peptidbindung R2-R3 imitieren die an APIasen binden und deren APIase-Aktivität dadurch inhibieren, wenn R2 für alle natürlichen Aminosäuren steht und R3 folgende Aminosäuren einschließt: Methionin, Alanin, Serin, Glutaminsäure, Leucin, Lysin, Isoleucin und Glycin.

Die Verbindungen der erfindungsgemäßen Arzneimittel schliessen Verbindungen ein, deren Bindungsmotif auf der einen Seite durch hydrophobe Gruppen und auf der anderen Seite des Bindungsmotifs durch hydrophobe oder positiv geladene Gruppen flankiert wird, wobei die flankierenden . Gruppen in elektrostatischen oder hydrophoben Kontakt zum katalytischen Zentrum der jeweiligen APIase stehen.

Die vorliegende Erfindung schließt insbesondere Peptide und Polypeptide als Inhibitoren von APIase-Aktivitäten ein. Die Peptide und Polypeptide vorliegender Erfindung sind natürlich vorkommende Aminosäuren (z.B.: L-Aminosäuren) und kleine Moleküle, welche biologisch und biochemisch die inhibierenden Peptide als sogenannte Peptidanaloga, Derivative oder Mimetika (Saragovi HU., et al., 1992, 10:773-778) simulieren können.

Die Polypeptid- oder Peptidinhibitoren vorliegender Erfindung können eine lineare oder zyklische Konformation besitzen. Verbindungen, die APIase inhibierende Aktivität besitzen, lassen sich mittels degenerierter Pepüdbibliotheken und dem hier beschriebenen APIase-Aktivitätsassay auffinden.

Die Verbindungen der erfindungsgemäßen Arzneimittel können zwischen 2 und 200 Aminosäuren lang sein. Vorzugsweise bestehen diese Inhibitoren aber aus 2 bis 20 Aminosäuresten und in einer besonderen Ausführungsform aus 3 bis 6 Resten. APIase-Inhibitoren einer besonders geeigneten Ausführungsform bestehen aus 4 Aminosäureresten, mit folgender Konsensussequenz: R¹-R²-(CONH)-R³-R⁴, in der R1, R2 und R4 jede natürliche L-Aminosäure sein kann und R3 ausschließlich für die L-Aminosäuren Methionin, Alanin, Serin, Glutaminsäure, Leucin, Lysin, Isoleucin und Glycin steht.

Die erfindungsgemäß verwendeten Peptide oder Peptidmimetika können mittels Standardmethoden synthetisiert werden, welche allgemein bekannt sind und Standardtechniken der Festphasensynthese einbezieht. Die Verbindungen, welche aus natürlichen Aminosäuren bestehen, lassen sich auch durch rekombinante DNA-Techniken herstellen. Die Inhibitoren dieser Erfindung sind entweder aus den 20 natürlich vorkommenden Aminosäuren oder anderen snthetischen Aminosäuren aufgebaut.

Synthetische Aminosäuren beinhalten zum Beispiel Naphthylalanin, L-Hydroxy-propylglycin, L-3,4-dihydroxy-phenylalanin und Aminosäuren wie L-alpha-hydroxy-lysin und L-alpha-methyl-alanin aber auch beta-Aminosäuren wie z.B.: beta-Alanin und Isoquinolin. Andere geeignete nicht-natürlichen Aminosäuren können solche sein, bei denen die normale Seitenkette der 20 natürlichen Aminosäuren durch andere Seitenketten ersetzt wurde, z.B. mit solchen Gruppen wie langkettige und kurzkettige Alkylreste, Zyklische 4-, 5-, 6-, bis 7-gliedrige Alkylringe, Amide, alkylierte Amide, alkylierte Diamide, kurzkettige Alkoxygruppen, Hydroxyl- und Carboxygruppen, sowie kurzkettige Ester und ihre Derivate oder 4-, 5-, 6-, bis 7-gliedrige Heterozyklen. Der Begriff kurzkettige Alkylreste bezeichnet lineare und verzweigte Ketten von Alkylgruppen mit 1 bis 6 Kohlenstoffatomen, wie Methyl, Ethyl, Propyl, Butyl usw.. Der Begriff kurzkettige Alkoxygruppen beschreibt lineare und verzweigte Ketten von Alkoxygruppen die aus 1 bis 16 Kohlenstoffatomen bestehen, wie z.B. Methoxy, Ethoxy usw.

Zyklische Gruppen können gesättigt oder ungesättigt sein. Die ungesättigten können aromatisch oder nicht-aromatisch sein.

C5 - bis C34-carbozyklische Strukturen umfassen hierbei gesättigte und ungesättigte mono- und bizyklische Verbindungen mit 5 bis 34 Kohlenstoffatomen, insbesondere Cyclopentan, Cyclohexan, Cycloheptan, Cyclooctan, Cyclononan, Cyclodecan, Cycloundecan, Cyclododecan Cyclopenten, Cyclohexen, Cyclohepten, Cycloocten, Cyclononen, Cyclodecen, Cycloundecen, Cyclododecen Cyclopentan, Cyclohexan, Cycloheptan, Cyclooctan, Cyclononan, Cyclodecan, Cycloundecan, Cyclododecan Cyclopenten, Cyclohexen, Cyclohepten, Cycloocten, Cyclononen, Cyclodecen, Cycloundecen, Cyclododecen, Bicyclohexan, Bicycloheptan, Bicyclooctan, Bicyclononan, Bicyclodecan, Bicycloundecan, Bicyclododecan, Bicyclohepten, Bicycloocten, Bicyclononen, Bicyclodecen, Bicycloundecen, Bicyclododecen, sowie C5-bis C34 Spiroverbindungen und kondensierte Ringsysteme wie z..B. Decalin, Hydrindan.

Erfindungsgemäß werden unter benzoiden bzw. nichtbenzoiden Aromaten die folgenden Gruppen verstanden: Benzol, Naphthalin, Cyclopentadien, Inden, Fluoren, Indan und Tetralin.
Ganz besonders bevorzugt hierbei Benzol und Naphthalin
Ferner können R2 und R3 gesättigte oder ungesättigte Isocyclen (a), wie (a1) monocyclische Verbindungen mit einer Ringgröße zwischen 5 und 7 Kohlenstoffatomen, wie (a2) Moleküle, die zwar mehrere, aber unabhängige Ringe im Molekül haben und entweder wie Biphenyl direkt aneinander gebunden oder wie das Diphenylmethan durch zwischengeschaltete Glieder miteinander verbunden sind, oder (a3) als Polycyclen wie das Inden (a31) o-kondensiert sind oder wie das Camphan (a32) mehr als zwei gemeinsame C-Atome in den Ringen haben oder (a33) bei denen wie beim Acenaphthen durch Perikondensation mehr als zwei C-Atome mehreren Ringen gemeinsam angehören oder (a34) bei denen wie bei den Spiranen je zwei Ringe ein gemeinsames quartäres C-Atom besitzen, wie auch Molekül (a4), die alicyclischer Natur sind und entweder zu den Monoterpenen mit der Summenformel C₁₀H₁₆₋₂₀ oder den bicylischen Terpenen, den Sesquiterpenen, den Diterpenen oder den Triterpenen zugerechnet werden, wobei der Grundkörper des Aminosäuremimetikums auch (a5) aromatisch sein kann und entweder aus einem Ring mit 6 Kohlenstoffatomen bestehen kann oder wie bei reinen Aromaten aus (a51) kondensierten Ringsystemen, (a52) nichtkondensierten cyclischen Ringsystemener, bzw. (a53) andere kondensierte Ringsysteme einschließt, sein.

Heterocyclische Gruppen haben insbesondere eine Ringgröße von 5 bis 8 Kohlenstoffatomen beinhalten typischerweise ein oder mehrere Heteroatome, wie Stickstoff, Sauerstoff, und/oder Schwefel, wie z.B. furazanyl, furyl, imidazolidinyl, imidazolyl, imidazolinyl, indolyl, isothiazolyl, isoxazolyl, morpholinyl (z.B.: morpholino), oxazolyl, piperazinyl (z.B. 1-piperazinyl), piperidyl (e.g. 1-piperidyl, piperidino), pyranyl, pyrazinyl, pyrazolidinyl, pyrazolinyl, pyrazolyl, pyridazinyl, pyridyl, pyrimidinyl, pyrrolidinyl (z.B.: 1-pyrrolidinyl), pyrrolinyl, pyrrolyl, thiadiazolyl, thiazolyl, thienyl, thiomorpholinyl (e.g. thiomorpholino) und triazolyl. Die heterozyklischen Gruppen können substituiert oder unsubstituiert sein. Wenn eine Gruppe substituiert ist, kann der Substituent Alkyl, Alkoxy, Halogen, Oxygen oder ein substituierter oder nichtsubstituierter Phenylrest sein (U.S. Patent No. 5,654,276 und U.S. Patent No. 5,643,873).

Unter dem Begriff "Aminosäuremimetika" wie in der vorliegenden Erfindung für R2 und R3 verwendet, werden Strukturen wie in sie in AU658636, CN1069735, AU1829792, EP0519640, CA2071061, US5422426, ZA9204244, IE7585892 beschrieben bzw. hergestellt werden verstanden. Vorschriften zu ihrer Herstellung findet man weiterhin in folgenden Dokumenten:
Hruby VJ. Slate CA. Amino acid mimetics and design of peptidomimetics for opioid and melanocortin receptors: General perspectives. [Article] ADVANCES IN AMINO ACID MIMETICS AND PEPTTDOMIMETICS, VOL 2, 1999. 2 PG. 191-220. 1999.,
Scholz D. Weber-Roth S. Macoratti E. Francotte E. Expedient synthesis of alpha-substituted alpha,beta-unsaturated gamma-amino acids (dipeptide mimetics);Wittig reaction of alpha-amino aldehydes with alpha-substituted alkoxycarbonyl phosphoranes. [Article] Synthetic Communications. 29(7):1143-1155, 1999.,
AMINO ACIDS AND PEPTIDES - LII - DESIGN AND SYNTHESIS OF OPIOID MIMETICS CONTAINING A PYRAZINONE RING AND EXAMINATION OF THEIR OPIOID RECEPTOR BINDING ACTIVITY. [Article] Chemical &Pharmaceutical Bulletin. 46(9):1374-1382,1998 Sep.,
Burger K. Mutze K. Hollweck W. Koksch B. INKORPORATION OF ALPHA-TRIFLUOROMETHYL SUBSTTTUTED ALPHA-AMINO ACIDS INTO C- AND N-TERMINAL POSITION OF PEPTIDES AND PEPTIDE MIMETICS USING MULTICOMPONENT REACTIONS. [Article] Tetrahedron. 54(22):5915-5928, 1998 . May 28.,
Hanessian S. Menaughtonsmith G. Lombart HG. Lubell WD. DESIGN AND SYNTHESIS OF CONFORMATIONALLY CONSTRAINED AMINO ACIDS AS VERSATILE SCAFFOLDS AND PEPTIDE MIMETICS [Review]. [Review] Tetrahedron. 53(38):12789-12854, 1997 Sep 22.,
Sabol JS. Flynn GA. Friedrich D. Huber EW. CONSTRAINED AMINO ACIDS - THE SYNTHESIS OF GLUTAMINE MIMETICS. [Article] Tetrahedron Letters. 38(21):3687-3690,1997 May 26.,
Moree WJ. Sears P. Kawashiro K. Witte K. Wong CH. EXPLOITATION OF SUBTILISIN BPN' AS CATALYST FOR THE SYNTHESIS OF PEPTIDES CONTAINING NONCODED AMINO ACIDS, PEPTIDE MIMETICS AND PEPTIDE CONJUGATES. [Article] Journal of the American Chemical Society. 119(17):3942-3947, 1997 Apr 30.,
Lombart HG. Lubell WD. RIGID DIPEPTIDE MIMETICS - EFFICIENT SYNTHESIS OF ENANTIOPURE INDOLIZIDINONE AMINO ACIDS. [Article] Journal of Organic Chemistry. 61(26):9437-9446, 1996 Dec 27.,
Reetz MT. Griebenow N. Goddard R. STEREOSELECTIVE SYNTHESES OF ALPHA-HYDROXY-GAMMA-AMINO ACIDS - POSSIBLE GAMMA-TURN MIMETICS. [Article] Journal of the Chemical Society - Series Chemical Communications. (16):1605-1606,1995 Aug 21.,
Liao, Subo. DESIGN AND SYNTHESIS OF TOPOGRAPHICALLY CONSTRAINED AMINO ACIDS, AND BIOACTIVE PEPTIDES FOR STUDIES OF LIGAND-RECEPTOR INTERACTION, AND FOR DE NOVO DESIGN OF DELTA-OPIOID SELECTIVE NON-PEPTIDE MIMETICS AS POTENTIAL THERAPEUTICS. Dissertation Abstracts International. Volume: 58-08, Section: B, page: 4232.,
Borg, Susanna. HETEROCYCLIC PEPTIDE MIMETICS DERIVED FROM BOC-AMINO ACIDS: DESIGN, SYNTHESIS AND INCORPORATION IN SUBSTANCE P AND DERMORPHIN. Dissertation Abstracts International. Volume: 57-02, Section: C, page: 0591.,
Nesloney, Carey Lynn. BIPHENYL-BASED UNNATURAL AMINO ACIDS DESIGNED TO NUCLEATE BETA-SHEET STRUCTURE AND PROGRESS TOWARDS TRIPHENYL-BASED NON-PEPTIDE ALPHA-HELIX MIMETICS. Dissertation Abstracts International. Volume: 56-10, Section:B, page: 5498.,
Markwell RE. Rahman SS. Ward RW. Synthesis of aminoazalactams as cyclic mimetics of basic alkyl amino acids. [Journal] BIOORG MED CHEM LETT, Vol 3(12) (pp 2537-2540), 1993.,
Rico JG. Synthesis of novel beta-amino acid precursors: beta-Amino-hydrocoumarins as unusual aspartic acid mimetics used in fibrinogen receptor.
Auf die vorstehend genannten Dokumente wird in ihrem ganzen Inhalt Bezug genommen.
Unter dem Begriff "Peptidmimetika" wie in der vorliegenden Erfindung für R2 und R3 verwendet, werden Strukturen wie in sie in Okada Y. Fukumizu A. Takahashi M. Yamazaki J. Yokoi T. Tsuda Y. Bryant SD. Lazarus LH. Amino acids and peptides. LVI. Synthesis of pyrazinone ring-containing opioid mimetics and examination of their opioid receptor binding activity. [Journal] Tetrahedron. Vol 55(50) (pp 1.4391-14406), 1999.
Andrade-Gordon P. Maryanoff BE. Derian CK. Hang H-C. Addo MF. Darrow AL. Eckardt AJ. Hoekstra WJ. McComsey DF. Oksenberg D. Reynolds EE. Santulli RJ. Scarborough RM. Smith CE. White KB. Design, synthesis, and biological characterization of a peptide-mimetic antagonist for a tethered-ligand receptor. [Journal] Proceedings of the National Academy of Sciences of the United States of America. Vol 96(22) (pp 12257-12262), 1999.
Okada Y. Fukumizu A. Takahashi M. Yamazaki J. Yokoi T. Tsuda Y. Bryant SD. Lazarus LH. Amino acids and peptides. LVI. Synthesis of pyrazinone ring-containing opioid mimetics and examination of their opioid receptor binding activity. [Journal] Tetrahedron. Vol 55(50) (pp 14391-14406), 1999.
Andrade-Gordon P. Maryanoff BE. Derian CK. Zhang H-C. Addo MF. Darrow AL. Eckardt AJ. Hoekstra WJ. McComsey DF. Oksenberg D. Reynolds EE. Santulli RJ. Scarborough RM. Smith CE. White KB. Design, synthesis, and biological characterization of a peptide-mimetic antagonist for a tethered-ligand receptor. [Journal] Proceedings of the National Academy of Sciences of the United States of America. Vol 96(22) (pp 12257-12262), 1999.
Li M. Lin Z. Johnson ME. Structure-based design and synthesis of novel thrombin inhibitors based on phosphinic peptide mimetics. [Journal] Bioorganic & Medicinal Chemistry Letters. Vol 9(14) (pp 1957-1962), 1999.
Boatman PD. Ogbu CO. Eguchi M. Kim H-O. Nakanishi H. Cao B. Shea JP. Kahn M. Secondary structure peptide mimetics: Design, synthesis, and evaluation of beta-strand mimetic thrombin inhibitors. [Journal] Journal of Medicinal Chemistry. Vol 42(8) (pp 1367-1375), 1999.
Deghenghi R. Synthetic peptides and their non-peptidyl mimetics in endocrinology: From synthesis to clinical perspectives. [Journal] Journal of Endocrinological Investigation. Vol 21(11) (pp 787-793), 1998.
Yang H. Sheng XC. Harrington EM. Ackermann K. Garcia AM. Lewis MD. Synthesis of sulfur-containing olefinic peptide mimetic farnesyl transferase inhibitors using the Nozaki-Hiyama-Kishi reaction and cuprate S(N)2' displacements. [Journal] Journal of Organic Chemistry. Vol 64(1) (pp 242-251), 1999.
Okada Y. Tsukatani M. Taguchi H. Yokoi T. Bryant SD. Lazarus LH. Amino acids and peptides. LII. Design and synthesis of opioid mimetics containing a pyrazinone ring and examination of their opioid receptor binding activity. [Journal] Chemical & Pharmaceutical Bulletin. Vol 46(9) (pp 1374-1382), 1998.
Kent DR. Cody WL. Doherty AM. The asymmetric synthesis of arginine mimetics: Derivatives of (S)-2-, 3- and 4-amidinophenylalanine suitable for incorporation into enzyme inhibitors and/or peptides. [Journal] Journal of Peptide Research. Vol 52(3) (pp 201-207), 1998.
Bisang C. Jiang L. Freund E. Emery F. Bauch C. Matile H. Pluschke G. Robinson JA. Synthesis, conformational properties, and immunogenicity of a cyclic template-bound peptide mimetic containing an NPNA motif from the circumsporozoite protein of plasmodium falciparum. [Journal] Journal of the American Chemical Society. Vol 120(30) (pp 7439-7449), 1998.
Burke TR Jr. Yao Z-J. Zhao H. Milne GWA. Wu L. Zhang Z-Y. Voigt JH. Enantioselective synthesis of nonphosphorus-containing phosphotyrosyl mimetics and their use in the preparation of tyrosine phosphatase inhibitory peptides. [Journal] Tetrahedron. Vol 54(34) (pp 9981-9994), 1998.
Tselios T. Probert L. Kollias G. Matsoukas E. Roumelioti P. Alexopoulos K. Moore GJ. Matsoukas J. Design and synthesis of small semi-mimetic peptides with immunomodulatory activity based on myelin basic protein (MBP). [Journal] Amino Acids. Vol 14(4) (pp 333-341), 1998.
Hanessian S. McNaughton-Smith G. Lombart H-G. Lubell WD. Design and synthesis of conformationally constrained amino acids as versatile scaffolds and peptide mimetics. Tetrahedron report number 426. [Journal] Tetrahedron. Vol 53(38) (pp 12789-12854), 1997.
Pfeifer ME. Linden A. Robinson JA. 111. Synthesis of a novel tricyclic dipeptide template and its incorporation into a cyclic peptide mimetic containing an NPNA motif. [Journal] Helvetica Chimica Acta. Vol 80(5) (pp 1513-1527), 1997.
Wipf P. Henninger TC. Solid-phase synthesis of peptide mimetics with (E)-alkene amide bond replacements derived from alkenylaziridines. [Journal] Journal of Organic Chemistry. Vol 62(6) (pp 1586-1587), 1997.
Lenman MM. Ingham SL. Gani D. Synthesis and structure of cis-peptidyl prolinamide nimetics based upon 1,2,5-äiazepine-3,6-diones. [Journal] Chemical Communications. Vol 1(1) (pp 85-87), 1996.
Qian Y. Vogt A. Sebti SM. Hamilton AD. Design and synthesis of non-peptide Ras CAAX mimetics as potent farnesyltransferase inhibitors. [Journal] Journal of Medicinal Chemistry. Vol 39(1) (pp 217-223), 1996.
Gramberg D. Weber C. Beeli R. Inglis J. Bruns C. Robinson JA. Synthesis of a type-VIbeta-turn peptide mimetic and its incorporation into a high-affinity somatostatin receptor ligand. [Journal] Helvetica Chimica Acta. Vol 78(6) (pp 1588-1606), 1995.
Olson GL. Cheung H-C. Chiang E. Madison VS. Sepinwall J. Vincent GP. Winokur A. Gary KA. Peptide mimetics of thyrotropin-releasing hormone based on a cyclohexane framework: Design, synthesis, and cognition-enhancing properties. [Journal] Journal of Medicinal Chemistry. Vol 38(15) (pp 2866-2879), 1995.
Gomez Monterrey IM. Gonzalez-Muniz R. Herranz R. Garcia-Lopez MT. Synthesis of 8-amino-3-oxoindolizidine-1-carboxylic acid derivatives as conformationally restricted templates for use in design of peptide mimetics. [Journal] Tetrahedron. Vol 51(9) (pp 2729-2736), 1995.
Dodd DS. Kozikowski AP. Cusack B. Richelson E. Synthesis of partially non-peptidic neurotensin mimetics. [Journal] BIOORG MED CHEM LETT, Vol 4(10) (pp 1241-1246), 1994.
Ripka WC. De Lucca GV. Bach AC II. Pottorf RS. Blaney JM. Protein beta-turn mimetics II: Design, synthesis, and evaluation in the cyclic peptide gramicidin S. [Journal] Tetrahedron. Vol 49(17) (pp 3609-3628), 1993.
Ripka WC. De Lucca GV. Bach AC II. Pottorf RS. Blaney JM. Protein beta-turn mimetics. I. Design, synthesis, and evaluation in model cyclic peptides. [Journal] Tetrahedron. Vol 49(17) (pp 3593-3608), 1993.
Nagai U. Sato K. Nakamura R. Kato R. Bicyclic turned dipeptide (BTD) as a beta-turn mimetic; its design, synthesis and incorporation into bioactive peptides. [Journal] Tetrahedron. Vol 49(17) (pp 3577-3592), 1993.
Currie BL. Krstenansky JL. Lin Z-L. Ungwitayatorn J. Lee Y-H. Del Rosario-Chow M. Sheu W-S. Johnson ME. Design and synthesis of a bicylic non-peptide beta-bend mimetic of enkephalin. [Journal] Tetrahedron. Vol 49(17) (pp 3489-3500), 1993.
Singh J. Gordon TD. Earley WG. Morgan BA. An efficient synthesis and acylation of alpha-amino-beta-keto-esters: Versatile intermediates in the synthesis of peptide mimetics. [Journal] Tetrahedron Letters. Vol 34(2) (pp 211-214), 1993.
Sato M. Lee JYH. Nakanishi H. Johnson ME. Chrusciel RA. Kahn M. Design, synthesis and conformational analysis of gamma-turn peptide mimetics of bradykinin. [Journal] Biochemical & Biophysical Research Communications. Vol 187(2) (pp 999-1006), 1992.
Krstenansky JL. Del Rosario-Chow M. Currie BL. The synthesis of syn- and anti-2(S)-phthalimidomethyl-2,3,4,4a,7,7a-hexahydro-6-oxo-5H-pyrano-[2 ,3-b]pyrroles as rigid beta-bend peptide-mimetics. [Journal] Journal of Heterocyclic Chemistry. Vol 29(4) (pp 707-711), 1992.
Gonzalez-Muniz R. Dominguez MJ. Garcia-Lopez T. Cativiela C. Garcia JI. Mayoral JA. Synthesis of 2-substituted 8-amino-3-oxoindolizidine-2-carboxylic acid derivatives as peptide conformation mimetics. [Journal] Tetrahedron. Vol 48(24) (pp 5191-5198), 1992.
Kahn M. Chen B. Methodology for the synthesis of mimetics of peptide beta-turns. [Journal] Tetrahedron Letters. Vol 28(15) (pp 1623-1626), 1987.
Kullmann W. Design, synthesis, and binding characteristics of an opiate receptor mimetic peptide. [Journal] Journal of Medicinal Chemistry. Vol 27(2) (pp 106-115), 1984 beschrieben werden.

Auf die vorstehend genannten Dokumente wird in ihrem ganzen Inhalt Bezug genommen.

Biologisch aktive Derivate oder Analoga der oben beschriebenen Inhibitoren, im weiteren als Peptidmimetika beschrieben, können nach dem Stand der Technik vom Fachmann hergestellt werden (U.S. Patent No. 4,612132, U.S. Patent No. 5,643,873, U.S. Patent No. 5,654,276). Diese Mimetika basieren auf einer spezifischen APIase-Inhibitor Sequenz unter Beibehaltung entsprechender Positionen bezüglich des zu Grunde liegenden Inhibitors. Diese Peptidmimetika besitzen biologische Aktivität (d.h. APIase inhibierende Aktivität), die vergleichbar der biologischen Aktivität des zu Grunde liegenden Inhibitors ist. Diese Peptidmimetika besitzen aber einen "biologischen Vorteil" gegenüber dem zu Grunde liegenden Peptidinhibitors bezüglich einem oder mehreren der folgenden Eigenschaften: Löslichkeit, Stabilität und Unempfindlichkeit gegenüber Hydrolyse und Proteolyse.

Methoden, solche Peptidmimetika herzustellen, schließen die Modifizierung der N-terminalen Amino Gruppe, der C-terminalen Carboxy-Gruppe und die Änderung von einer oder mehreren Peptidbindungen des Peptids zu nicht-peptidischen Bindungen ein. Dabei kann es vorteilhaft sein, zwei oder mehrere solche Modifizierungen zu Herstellung eines solchen Peptidmimetika vorzunehmen. Im folgenden sind Beispiele von Modifikationen von Peptiden aufgeführt, die zur Herstellung von Peptidmimetika führen. Die entsprechenden Techniken sind z.B. in US Patent 5,643,873 und US Patent 5,654,276 beschrieben. Diese Techniken werden genutzt um Mimetika von APIase-Inhibitoren herzustellen.

***Modifikationen des N-Terminus:*** Nach Festphasensynthese des Peptidinhibitors, wird die N-terminale Schutzgruppe selektiv so entfernt, dass alle anderen funktionellen Gruppen weiterhin geschützt bleiben und das Molekül über den C-terminus weiterhin mit der festen Phase verbunden bleibt. Dadurch wird es möglich den N-terminus des Peptides so zu verändern, dass er dem gewünschten Mimetika entspricht.

Modifikationen des N-Terminus schließen ein: Alkylierung, Azetylierung, Addition einer Carbobenzoylgruppe, Ausbildung eines Succininidrestes usw. Im einzelnen kann die N-terminale Aminogruppe wie im folgenden dargelegt, umgesetzt werden:

a)Mit einem Säurehalogenid (z.B., RC(O)Cl) oder Säureanhydrid zu einer Amidgruppe der Formel RC(O)NH - worin R dem oben Definierten enspricht. Die Reaktion wird typischerweise mit äquimolaren Mengen oder einem Überschuß (z.B. ca 5 Äquivalenten) des Säurehalogenids zum Peptid in einer inerten Lösung (z.B. Dichlormethan) durchgeführt, welche vorzugsweise einen Überschuß (z.B. ca 10 Äquivalente) eines tertiären Amins, wie z.B. Düsopropylethylamin, zugesetzt wird, um die während der Reaktion gebildete Säure abzufangen. Konventionelle Reaktionsbedingungen, wie z.B. Raumtemperatur für 30 min, reichen im allgemeinen aus. Die aufgeführte Reaktion ist auch geeignet um N-Alkylamide der allgemeinen Formel RC(O)NR herzustellen.

b)Mit Bernsteinsäureanhydrid zur Succinimidgruppe. Wie oben angegeben, sind entweder äquimolare Mengen oder ein Überschuß des Anhydrids (z.B. ca 5 Äquivalente) nötig, um die N-terminale Aminogruppe in eine Succinimidgruppe zu überführen. Die verwendete Methodik ist allgemein bekannt, wie auch die Anwendung eines Überschusses (z.B. 10 Äquivalente) eines tertiären Amines, wie z.B. Diisopropyethylenamin in einem gebräuchlichen inerten Lösungsmittel (z.B. Dichlormethan) und wird z.B. im US Patent 4,612,132 beschrieben, einschließlich zahlreicher Literaturreferenzen. Die Succinimidgruppe selbst kann substituiert sein, so z.B. mit C₂-C₆ Alkylresten oder -SR Substitutienten welche mit dem Fachmann bekannten Methoden hergestellt werden können. Solche Alkylsubstitutienten lassen sich z.B. wie im im US Patent 4,612,132 beschrieben durch eine Maleinsäureanhydrid-Mehode herstellen, ebenso die entsprechenden -SR Verbindungen durch die Reaktion von RSH mit Maleinsäureanhydrid, wobei R weiter oben definiert wurde.

c) zu einem benzyloxycarbonyl-NH oder einer substituierten benzyloxycarbonyl-NH-Gruppe, durch Reaktion mit etwa einem Äquivalent oder einem Überschuß von CBZ-Cl (d.h. Benzyloxycarbonylchlorid) oder einem substituierten CBZ-Cl in einem üblichen inerten Lösungsmittel (wie z.B. Dichloromethan), welches vorzugsweise ein tertiäres Amin enthält um die während der Reaktion gebildete Säure abzufangen.

d) zu einer Sulfonamidgruppe, durch Reaktion mit einer äquivalenten Menge oder einem Überschuß (z.B. 5 Äquivalente) von R-S(O)₂CL in einer gebräuchlichen inerten Lösungsmittel (wie z.B. Dichloromethan). Vorzugsweise enthält das inerte Lösungsmittel einen Überschuß an tertiärem Amin (z.B. 10 Äquivalente) wie z.B. Diisopropylethylamin um die während der Reaktion gebildete Säure abzufangen. Die Reaktionsbedingung sind dem Fachmann bekannte Standardbedingungen, wie z.B. Raumtemperatur und eine Reaktionszeit von ca 30 min.

e) zu einer Carbamatgruppe, durch Reaktion mit einer äquivalenten Menge oder einem Überschuß (z.B. 5 Äquivalente) von R-OC(O)CL oder R-OC(O)C₆H₄-p-NO₂ in einer gebräuchlichen inerten Lösungsmittel (wie z.B. Dichloromethan). Vorzugsweise enthält das inerte Lösungsmittel einen Überschuß an tertiärem Amin (z.B.etwa 10 Äquivalente) wie z.B. Diisopropylethylamin um die während der Reaktion gebildete Säure abzufangen. Die Reaktionsbedingung sind dem Fachmann bekannte Standardbedingungen, wie z.B. Raumtemperatur und eine Reaktionszeit von ca 30 min; und

f) zu einem Harnstoffgruppierung der allgemeinen Formel RNHC(O)NH- durch Reaktion der terminalen Aminogruppe mit einer äquivalenten Menge oder einem Überschuß (z.B. 5 Äquivalenten) von R-N=C=O in einem gebräuchlichen inerten Lösungsmittel (wie z.B. Dichloromethan), wobei R oben definiert wurde. Vorzugsweise enthält das inerte Lösungsmittel einen Überschuß an tertiärem Amin (z.B.etwa 10 Äquivalente) wie z.B. Düsopropylethylamin um die während der Reaktion gebildete Säure abzufangen. Die Reaktionsbedingung sind dem Fachmann bekannte Standardbedingungen, wie z.B. Raumtemperatur und eine Reaktionszeit von ca 30 min.

***Modifizierung des C-Terminus:*** Zur Herstellung von Peptidmimetika, in denen die C-terminale Cyrboxylgruppe durch einen Ester ersetzt ist (d.h. -C(O)OR; R wurde weiter oben definiert) werden entsprechende dem Fachmann bekannte Syntheseharze verwendet. Die mit Schutzgruppen versehene Verbindung kann unter basischen Bedingungen und Alkohol (z.B. Methanol) vom Harz abgelöst werden. Der gewünschte Ester kann dann in üblicher Weise von seinen Schutzgruppen (z.B. durch Versetzen mit HF) befreit werden um den gewünschten Ester zu erhalten.

Zur Herstellung eines Peptidmimetikas, in welchem die C-terminale Gruppierung durch das Amid -C(O)NR³R⁴ ersetzt wurde, wird ein Benzhydrylaminharz als Trägermaterial für die Peptidsynthese verwendet. Nach Syntheseende führt das Versetzen mit HF direkt zur Freisetzung des freien Peptidamides (d.h. der C-Terminus wird zu -C(O)NH₂). Alternativ wird bei der Benutzung von chloromethylierten Harzen zum Erhalt des freien Peptidamids, das Syntheseproduktes vom Harz durch Ammoniak abgespalten. Wird die Abspaltung mit Alkylamin oder Dialkylamin durchgeführt, kann das Seitenketten geschützte Alkylamid oder Dialkylamid (d.h. der C-terminus ist -C(O)NR¹R², wobei R¹ und R² oben definiert wurden) erhalten werden. Der Schutz der Seitenkette kann dann wiederum, wie oben beschrieben durch Versetzen mit HF entfernt werden, um die gewünschten freien Amide, Alkylamide oder Dialkylamide zu erhalten.

Alternativ kann die C-terminale Carboxylgruppe oder der C-terminale Ester unter Entfernen der Hydroxyl- (-OH) oder der Ester- (-OR) Gruppe mit der N-terminalen Aminogruppe reagieren um ein zyklisches Peptid zu erhalten. Zum Beispiel kann nach erfolgreicher Synthese und Erhalt der freien Säure des gewünschten Peptides, dessen freie Säure mit einem geeigneten Carboxylgruppenaktivator, wie z.B. Dicyclohexylcarbodiimid (DCC) unter Verwendung entsprechender Lösungsmittel, wie z.B. in Mischungen mit Methylenchlorid (CH₂Cl₂), oder Dimethylformamid (DMF) in einen aktivierten Ester überführt werden. Zur Unterdrückung von Polymerisationsprodukten bei der spontanen Zyklisierung kann mit verdünnten Lösungen des Peptids gearbeitet werden. Die beschriebene Methode ist als eine der Standardmethoden dem Fachmann bekannt.

***Einführung nicht peptidischer Bindungen:*** Peptidmimetika in denen eine oder mehrere der Peptidbindungen [-C(O)NH-] ersetzt wurden durch solche Bindungen wie -CH₂-Carbamat-Bindung, Phosphonat Bindung, -CH₂-Sulfonamid Bindung, Urea-Bindung, sekundäre Amin (-C₂NH-) Bindung, oder eine alkylierte Peptidyl Bindung [-C(O)NR⁶ - worin R⁶ einem niedrigen Alkylrest entspricht] können durch konventionelle Synthese durch ledigliches Austauschen der zu ersetzenden Aminosäure durch das geeignete geschützte Aminosäureanalogon an der entsprechenden Stelle der Synthese erhalten werden.

Geeignete Verbindungen sind z.B. Aminosäureanaloga in denen die Carboxylgruppe der Aminosäure mit solch einer funktionellen Gruppe ersetzt wurde, welche geeignet ist eine der oben beschriebenen Bindungen auszubilden. Zum Beispiel: wenn die Peptidbindung - C(O)NR- in einem Peptid durch eine -CH₂-Carabamat Bindung (-Ch₂OC(O)NR-) ersetzt werden soll, wird die Carboxylgruppe (-COOH) einer entsprechend geschützten Aminosäure erst zu einer CH₂OH - Gruppe reduziert und mittels konventioneller Methoden zu einer -OC(O)Cl oder einer para-Nitrocarbonat -OC(O)O-C₆H₄-p-NO₂ Funktionalität überführt. Die Reaktion einer solchen funktionellen Gruppe mit dem freien Amin oder einem am N-Terminus alkylierten Amin eines partiell synthetisierten Peptids an der Festphase führt zu Bildung einer CH₂OC(O)NR-Bindung im synthetisierten Peptid.

In ähnlicher Weise lassen sich oben erwähnte Bindungen nach dem Stand der Technik in die zu synthetisierende Verbindung einführen. Zum Beispiel kann eine Peptidbindung durch eine CH₂-Sulfonamid Bindung auf folgende Weise ersetzt werden: Nach Reduktion der Carboxylgruppe (-COOH) einer geeigneten geschützten Aminosäure zu einer - CH₂OH-Gruppe, wird die Hydroxylgruppe genutzt um z.B. durch Umsetzung mit Toluol-4-sulfonylchlorid nach Standardmethoden einen Tosylrest einzuführen. Die Reaktion der tosylierten Verbindung mit z.B. Thioessigsäure und anschließender Hydrolyse und Sulfochlorierung führt zum -CH₂-S(O)₂Cl Rest, welcher die Carboxylgruppe ersetzt. Das Verwenden des so hergestellten Aminosäureanalogons in der Peptidsynthese führt zur Herstellung eines Peptidmimetika, bei dem eine Peptidbindung durch eine - CH₂S(O)2NR-Bindung ersetzt wurde.

In ähnlicher Weise lassen sich -CH₂NH-Bindungen an Stelle einer Peptidbindung nach dem Stand der Technik in die zu synthetisierende Verbindung bei Verwendung eines geeigneten Dipeptidanaloga, bei dem die Carbonylgruppe der Peptidbindung zu einer CH₂ -Gruppe mittels konventioneller Methoden umgewandelt wurde, einführen. Zum Beispiel liefert im Falle von Diglycin, die Reduktion des Amids das entsprechende Amin H₂NCH₂CH₂NHCH₂COOH, welches als N-geschütztes Derivat für den nächsten Syntheseschritt der Peptidsynthese von Peptidmimetika genutzt werden kann.

Die Substitution von Aminosäuren durch solche Aminosäureanaloga während der Synthese von Peptidmimetika kann während eines jeden solchen Syntheseschrittes getätigt werden, so dass auch Mimetika erhalten werden können, in denen alle oder nur einige der Peptidbindungen durch nicht peptidische Bindungen ersetzt sein können.

Inhibitoren vorliegender Entdeckung können ebenso zyklische Peptide und zyklische Peptidmimetika sein. Solche zyklischen Inhibitoren lassen sich mittels allgemein bekannter Techniken die z.B. in US Patent 5,654,276 oder USSN 08/864,392 (28. Mai 1997) beschrieben sind herstellen.

Erfindungsgemäße Arzneimittel umfassen somit hierbei Peptide oder Peptidmimetika mit der allgemeinen Grundstruktur

Y-R₂-R₃

wobei Y eine gesättigte oder ungesättigte, gerad- oder verzweigtkettige Fettsäure ist, die mit der Aminosäure oder dem Aminosäuremimetikum R₂ über eine C(O)NH-Bindung verknüpft ist, und wobei die Aminosäure oder das Aminosäuremimetikum R₃ über seine Aminogruppe mit R₂ über eine C(O)NH-Bindung verknüpft ist wobei R₂ alle natürlichen Aminosäuren oder ihre Mimetika und R₃ die Aminosäuren Methionin, Alanin, Serin, Glutaminsäure, Leucin, Lysin, Isoleucin und/oder glycin oder ihre Mimetika umfasst und gegebenenfalls einen pharmazeutisch verträglichen Träger.

Besonders bevorzugte erfindungsgemäße Arzneimittel umfassen hierbei Verbindungen der folgenden Formeln: 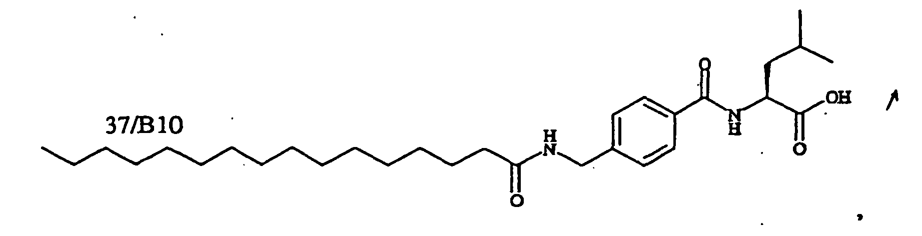 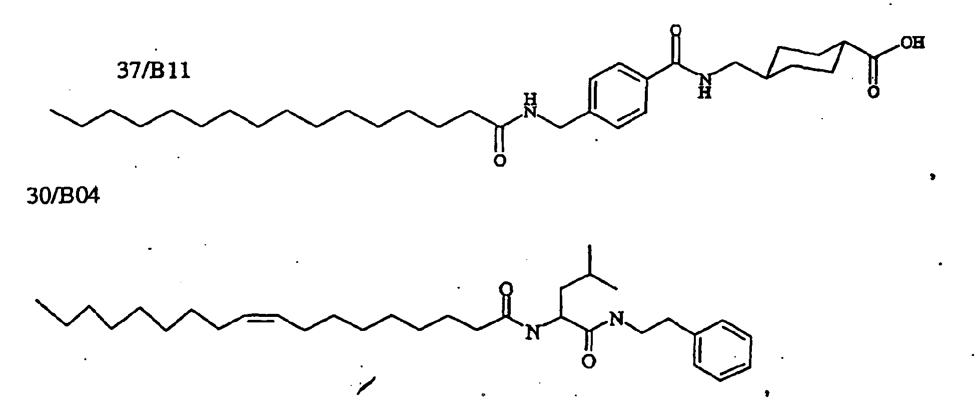 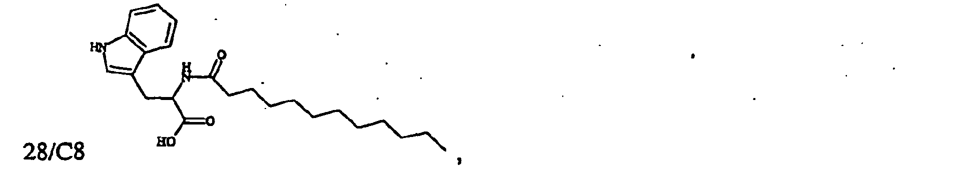 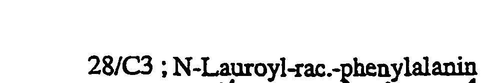 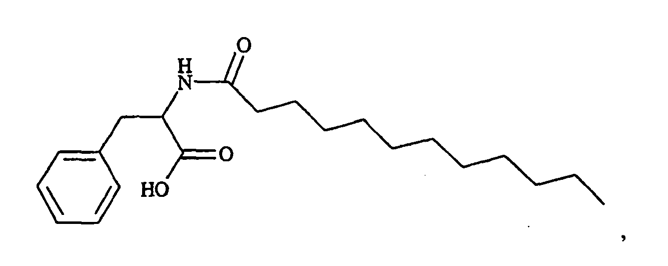 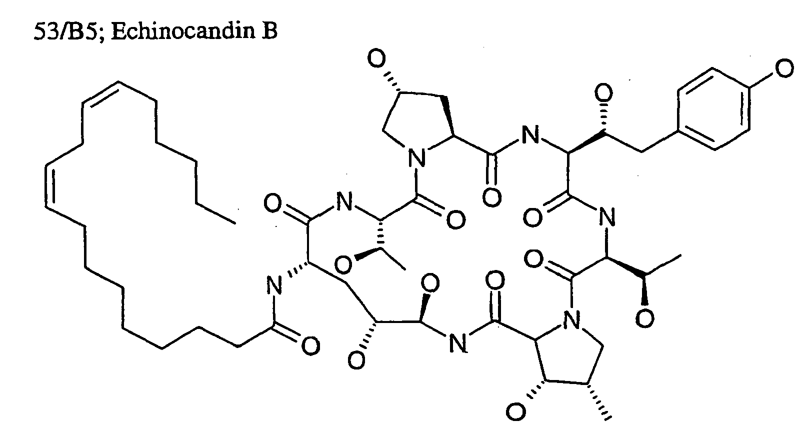 und

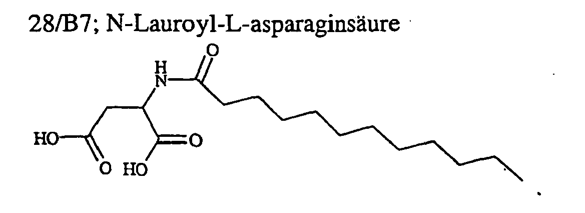

Ganz besonders bevorzugt ist hierbei

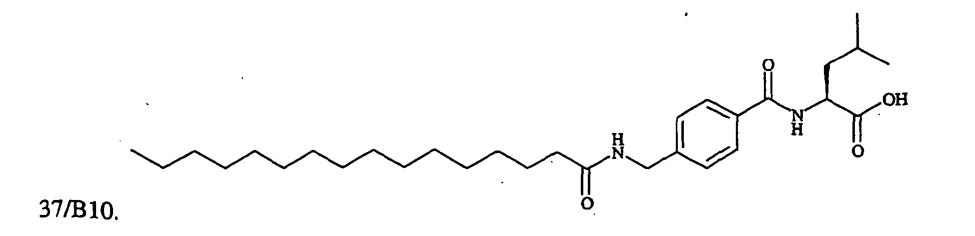

Die biologische Aktivität von Inhibitoren vorliegender Entdeckung kann wie oben angegeben, mittels ihrer Wirkung auf die APIase-Aktivität in entsprechenden APIase-Aktivitätsassays gefunden werden. Inhibitoren haben typischerweise eine Inhibitorkonstante (Kᵢ) die im nanomolaren Bereich oder darunter aber mindestens kleiner als 100 mikromolar, und noch mehr bevorzugt kleiner als 10 mikromolar ist. Inhibitorkonstanten lassen sich mit Methoden bestimmen, wie sie allgemein bekannt sind. So beinhaltet der IC₅₀-Wert als Inhibitionskonstante diejenige molare Konzentration eines Inhibitors, die benötigt wird, um die unter Standardbedingungen gemessene Aktivität eines Enzymes um 50 % zu vermindern.

Erfindungsgemäße Inhibitoren können *in vitro* angewendet werden um den Zellzyklus, die Teilung von Zellen (Mitose) aber auch die durch intrazelluläre oder extrazelluläre Signale ausgelöste Synthese von Proteinen zu untersuchen. Zum Beispiel können erfindungsgemäße Inhibitoren durch Inhibition einer spezifischen APIase genutzt werden um die dadurch bewirkten Effekte auf den Zellzyklus, die Mitose oder Proteinsynthese aufzuklären.

Erfindungsgemäße Inhibitoren können auch dazu genutzt werden um die Teilung von Zellen zu beeinflussen. So kann z.B. das Zellwachstum durch diese Inhibitoren beeinflusst werden. Erfindungsgemäße Inhibitoren der APIase-Aktivität können geeignet sein, um Zielzellen abzutöten. Sie können auch verwendet werden um als Wirkstoffe in der Behandlung von Infektionen durch Pilze oder Hefen, einschließlich mit Aspergillus, sowie - mit Parasiten (z.B. Malaria) bei Säugetieren angewendet zu werden. Der Begriff Säugetiere soll sich dabei auch auf Haustiere und Menschen beziehen.

Zum Beispiel ist die APIase-Aktivität von DnaK wichtig für die korrekte Faltung von Proteinen. Diese korrekte Faltung ist wiederum eine der Voraussetzungen für unterschiedlichste biochemische Abläufe in der Zelle, wie z.B. Mitose oder Apoptose. Deshalb führt die Inhibition z.B. von DnaK mit einem erfindungsgemäßen Inhibitor seiner APIase-Aktivität zum mitotischen Arrest der Zelle und nachfolgend zur Apoptose. Auf diese Art und Weise können APIase-Inhibitoren als therapeutische Wirkstoffe zur Beeinflussung von neoplastischen und hyperplastischen Krankheiten genutzt werden.

Neoplastische und Hyperplastische Krankheiten schließen dabei alle Formen von Wucherungen, Psoriäsis, Retinosis, Atherosklerose die von einer Plaque-Bildung verrührt, Leukämien und gutartiges Geschwülste ein. Auch solche Erkrankungen, wie Lymphome, Papilome, Lungenfibrose, Rheumatoid Arthritis und Multiple Sklerosis.

Die Hemmung der APIase-Aktivität kann insbesondere bei solchen Krankheitsprozessen von Vorteil sein, bei denen es über die Bildung falsch-gefalteter Proteine zu krankmachenden Veränderungen kommt. Solche Veränderungen werden neben den oben beschriebenen Krankheiten auch bei Krankheiten beobachtet, die mit massiven pathobiochemischen Strukturänderungen von Proteinen einhergehen, zu nennen sind hier Krankheiten wie Cystische Fibrose, Juveniles Lungenemphysem, Tay-Sachs Syndrome, Kongenitale Sucrose Isomaltase Defizienz oder Familiäre Hypercholesterolaemie oder transmissible spongiforme Encephalopathie (Prion-Krankheiten). Die Verabreichung von APIase-Inhibitoren kann einen solchen Einfluß auf diese Krankheiten ausüben, der von der Verlangsamung bis zur Remission des Krankheitsprozesses führen kann. Dabei betrifft die vorliegende Erfindung insbesondere Arzneimittel zur Verwendung bei der Behandlung oder Vorbeugung der vorstehenden Krankheiten. ,

APIase Inhibitoren können auch effektiv den Verlauf von bakteriellen Infektionen positiv beeinflussen, weil bekannt ist, daß APIasen wie DnaK für die Proteinbiosynthese der Bakterien notwendig sind (Deuerling E. et al., 1999, Nature 400:693-6; Teter SA., et al., 1999: Cell. 97:755-65).

Das Arzneimittel, das einen solchen erfindungsgemäßen Inhibitor enthält kann intravenös, parenteral, oral, durch Inhalation, mittels medizinischer Pflaster oder in Form von Suppitorien wie z.B. rektal einzuführende Zäpfchen medizinisch angewendet werden. Das Arzneimittel wird entweder als einmalige Dosis verabreicht oder aber in mehreren Dosen über eine solche Zeltspanne, die ausreichend ist um eine solche Wirkstoffkonzentration zu erreichen, die den gewünschten therapeutischen Effekt bewirkt.

Mögliche Pharmazeutische Träger schließen Stoffe ein, wie z.B.: Wasser, Salzlösungen, Alkohole, Polyethylenglykol, Gelatine, Kohlenhydrate wie Laktose, Amylose oder Stärke, Magnesiumstearate, Talk, Paraffinöl, Fettsäureester, Hydroxymethylzellulose, Plyvinylpyrolidone, usw., sind aber nicht darauf begrenzt. Es kann notwendig sein, die pharmazeutische Mischung zu sterilisieren und mit weiteren solchen Zusatzstoffen zu mischen, wie z.B. Gleitmittel, Konservierungsmittel, Stabilisierungsmittel, Benetzungsmittel, Emulgatoren, Salzen um den osmotischen Druck zu beeinflussen, Puffern, Farbstoffen und/oder Aromazusätzen, welche die aktive biologische Verbindung nicht oder nur wenig nachteilig beeinflussen. Vorteilhaft kann auch sein, die pharmazeutische Mischung mit weiteren Wirkstoffen, wie z.B. Inhibitoren proteolytischer Enzyme zu versetzen, um den Abbau des erfindungsgemäßen Inhibitors zu verzögern oder zu verhindern.

Bei parenteraler Verabreichung kann es von Vorteil sein, die injizierbare, sterile Lösung als ölige oder wässrige Lösung oder als Suspension bzw. Emulsion zu verwenden. Die verwendete Menge des Inhibitors für eine spezielle therapeutische Anwendung kann auch beeinflusst werden, durch die Art der Verabreichung, die Art der Zusammensetzung (Formulierung des Wirkstoffes) aber auch den individuellen Eigenschaften des Patienten, wie z.B. dem Alter, Körpergewicht oder Allgemeinzustand. So ist eine effektive Menge des Inhibitors eine solche Menge, die ausreichend ist, die gewünschte APIase-Aktivität so zu inhibieren, dass die beabsichtigten biologischen Effekte wie z.B. die gezielte Beeinflussung des Zellwachstums bestimmter Zellen ausgelöst wird. Wirksame Dosen für ein bestimmtes Individium werden gemäß der üblichen Praxis nach allgemeinen Erwägungen (z.B. mittels eines üblichen pharmakologischen Protokolls) festgelegt.

Weiterer Gegenstand der Erfindung ist die Bereitstellung einer Methode, die es ermöglicht, die Katalyse der *cis*/*trans*-Isomerisierung von sekundären-Amid Peptidbindungen durch spezifische Katalysatoren, den APIasen und ihren niedennolekularen Mimetika, nachzuweisen. Durch die überraschende Entdeckung, dass die cis/trans-Isomerisierung von sekundären-Amid Peptidbindungen durch APIasen spezifisch beschleunigt werden kann, ergibt sich die Möglichkeit, Bewertungsverfahren der Geschwindigkeit dieser Isomerisierung zu nutzen um die spezifische Katalyse durch Katalysatoren nachzuweisen. Die verwendeten Methoden die erfindungsgemäß genutzt werden können, schließen direkte Nachweisverfahren der Isomerisierung, wie z.B. NMR-Methoden (Beispiel 8) oder spektroskopische Nachweisverfahren (Beispiel 1-4,6-7), aber auch indirekte Verfahren, die nachgelagerte biochemische Reaktionen nutzen, wie z.B. Proteinfaltung, Hydrolyse oder isomerspezifische chemische Reaktionen (wie z.B. Phosphat-Transferreaktionen) ein. Durch nachgelagerte oder in Konkurrenz zur *cis*/*trans-*Isomerisierung einer kritischen sekundären-Amid Peptidbindung des Substrates ablaufenden, biochemische Reaktionen kann es zu messbaren Unterschieden in der Bildungsgeschwindigkeit der End-oder Zwischenprodukte dieser Reaktionen kommen: Solche quantifizierbaren physikalischen, chemischen, biochemischen oder biologischen Unterschiede können z.B. elektrophoretische Wanderungsgeschwindigkeiten, die Ausbildung eines Fluoreszenz- oder UV/Vis-Absorptionssignal, die Erkennung mittels Antikörpern oder der Transport durch biologische Membranen sein.

Die vorstehende Methode ermöglicht das Auffinden neuer Katalysatoren mit APIase-Aktivität in biologischen Materialien. Aus Isolationsexperimenten von APIasen aus biologische Materialine ergeben sich Hinweise auf weitere existierende Katalysatoren, da das Elutionsmuster typisch für ein Gemisch aus mehr als einer katalytisch aktiven Spezies ist.

Weitere Vorteile durch die Anwendung der katalysierten *cis*/*trans*-Isomerisierung von sekundären-Amid Peptidbindungen in Polypeptiden durch APIasen ergeben sich in der Biochemie/Biotechnologie. So ist die biochemisch/biotechnologische Herstellung von Proteinen und anderen Polypeptiden eine wesentliches Merkmal dieser Bereiche (Yon JM., 2001, J. Med.&Biolog. Res. **34**:419-435). Große Schwierigkeiten bei der ökonomischen Herstellung liegen oftmals in dem als Proteinfaltung bezeichneten Prozeß. Dieser Prozeß beinhaltet signifikante Änderungen der Winkel omega, phi und psi des Peptidkettenrückgrates. Findet eine Proteinfaltung statt, führt diese Faltung zu mindestens einer neuen qualitativen Eigenschaft des Moleküls, welches mindestens eine der in ihrer räumlichen Struktur geänderten Peptidbindung aufweist. Diese neue Qualität kann z.B. bei Enzymen die Ausbildung einer vom Fachmann als "aktives Zentrum" bezeichneten funktionellen Struktur sein oder eine bestimmte Substratspezifität (Shinde U. et al., 1999, J. Biol. Chem. 274:15615-15621), die neue Qualität kann aber auch oft ein nur schwierig mittels chemischer, biochemischer, biologischer oder physikalischer Methoden erfassbarer Unterschied sein. So können Chargen biotechnologisch hergestellter Proteine, deren funktionelle Gegebenheiten messbar sind, wie z.B. Enzyme, Rezeptorproteine, Inhibitoren oder Hormone, die oft mit einer Vielzahl dem Fachmann geläufiger Methoden wie Chromatographie, Massenspektrometrie, Aminosäureanälyse oder Circulardichroismus keine Unterschiede zeigen, mittels dieser funktionellen Gegebenheiten (wie spezifischer Aktivität oder Titerkonzentration) differenziert werden. Daneben gibt es eine ganze Reihe von Beispielen, bei denen die strukturellen Unterschiede so massiv sind, dass sie mit relativ groben Methoden, wie z.B. an Löslichkeitsunterschieden erkannt werden können. So führt ein als Aggregation bezeichneter Prozeß bei der Faltung von Proteinen in der Zelle zur Bildung von in wässrigen Lösungen nur schwer löslichen Inklusionskörpern (englisch: "Inclusionbodies") (Kopito RR., 2000 Trends in Cell Biology 10:524-530).

Die strukturellen Unterschiede der räumlichen Struktur von Peptidketten können aber auch darin bestehen, dass die eine Qualität im Gegensatz zur anderen aus Populationen weitgehend ungeordneter Strukturen (engl. Random coil) (Serrano L. Advances in Protein Chemistry 53:49-85 (2000)) besteht.

Unabhängig von der Quantität der räumlichen Strukturunterschiede von Peptidbindungen ist es oft vorteilhaft, den Prozess der Herstellung von Biomolekülen (Proteinen, Polypeptiden oder Oligopeptiden) mit den gewünschten Eigenschaften durch die Beteiligung unterschiedlichster Faltungshelfer zu unterstützen (z.B.: Stoller et al., 1995, EMBO J. 14:4939-4949 ; Buchner J., 1999, Trends in Bioch. Sciences 24:136-141; Mayer M. et al., 2000, J. Biol. Chem. 275:29421-29425; Schiene-Fischer C and Yu C., 2001, FEBS Letters 495:1-6).

Durch die Bereitstellung einer neuen Art von Faltungshelferkatalyse, diejenige die z.B. im aktiven Zentrum der APIasen realisiert wird, lässt sich das Repertoire welches dem Biotechnologen zu Verfügung steht, um durch die Verwendung von Faltungshelfern eine wirtschaftliche Herstellung von Biomolekülen zu ermöglichen, ergänzen. Dabei wird die Faltungshelferkatalyse entweder durch APIasen bzw.. ihre Varianten oder durch chemosynthetisch hergestellte niedermolekulare Mimetika der APIasen ausgeführt. Dabei kann es für die biotechnologische Prozessführung von Vorteil sein, unterschiedlichste Faltungshelfer in Lösung oder matrixgebunden, gleichzeitig oder in nachfolgenden Schritten oder aber auch in Kompartimenten getrennt, anzuwenden. Die biotechnologische Prozessführung der ökonomischen Herstellung bestimmter Biomoleküle unter Beteiligung von Faltungshelfern kann, dies ist dem Fachmann geläufig, *in vitro* aber auch *in vivo* ablaufen. Im Unterschied zum *in vitro* ablaufenden Prozess werden bei der Prozessführung *in vivo* solche biotechnologisch veränderten Zellen verwendet, die das oder die benötigten Faltungshelfer in der für die Katalyse des Faltungsprozeß notwendigen Menge enthalten.

### Beispiel 1: Die Geschwindigkeit der cis/trans-Isomerisierung der Peptidbindung des Substrates Ala-Leu lässt sich durch das Protein DnaK beschleunigen.

Die Peptidbindung des Substrates Ala-Leu befindet sich bei pH 7.4 zu etwa 99% in der trans-Konformation. Eine Erhöhung der Protonenkonzentration der wässrigen Ala-Leu - Lösung auf etwa 100 mM bewirkt eine Verschiebung des Gleichgewichtes der Konformationen zu höherem Gehalt des Peptides mit der cis-Konformation. Diese Verschiebung ist im Bereich von 2220-228 nm spektroskopisch messbar, da das Konformere mit der cis-Konformation bei dieser Wellenlänge einen geringeren Extinktionskoeffizienten als das Konformere mit der trans-Konformation aufweist. Die Geschwindigkeit der Isomerisierung wird einer Messung zugänglich, wenn das Substrat einer solchen pH-Änderung unterworfen wird, die einen Einfluß auf das *cis*/*trans*-Gleichgewichtes des Substrates bewirkt und wenn die Geschwindigkeit der pH-Änderung schneller als die Geschwindigkeit der Isomerisierung des Substrates ist. Dabei entspricht die Geschwindigkeit der Isomerisierung der Summe der Einzelgeschwindigkeiten *cis* nach *trans* und *trans* nach *cis* der beiden Konformere des Substrates. Im vorliegendem Beipiel wird eine sogenannte "Stopped-Flow" - Apparatur der Firma Applied Photophysics Ltd. (England) genutzt. Dieses spektroskopische Gerät ermöglicht eine schnelle Mischung zweier Komponenten und nachfolgend eine schnelle und zeitabhängige Sammlung spektroskopischer Daten. Eine zugehörige Software erlaubt darüber hinaus das Berechnen der Geschwindigkeitskonstanten (k_{obs}) bei Annahme eines Konzentrations/Zeit - Gesetzes, welches einem sog. "Erster Ordnungs Zeitgesetz" entspricht. Zum Nachweis der Katalayse wurde folgender Ansatz gewählt:
Stammlösung Ala-Leu (Bachem, Schweiz) 10 mM gelöst in Aqua dest. bei pH 2.0, eingestellt mittels HCl
Pufferlösung: 12.5 mM Tris, 50 mM KCl, 11 mM MgCl₂ mit HCl auf pH 8.8 justiert
DnaK (SIGMA-ALDRICH CHEMIE GmbH, Deutschland), bzw. molekularbiologisch hergestellt
Nach Vermischen der Komponenten in der Stopped-Flow Apparatur hatte die
Reaktionslösung folgende Konzentrationen: 1.66 mM Ala-Leu, 10.4 mM Tris, 41.6 mM KCl, 9.2 mM MgCl₂. Der pH betrug 7.6. Alle Lösungen wurden konstant bei einer Temperatur von 25 °C gehalten.

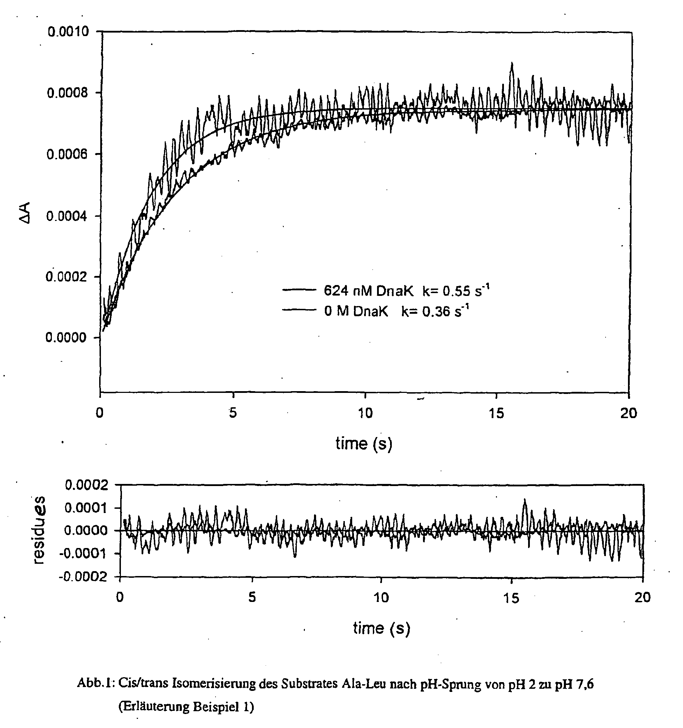

Die Abbildung 1 zeigt ein typisches Bild. Die untere Kurve, die erst nach ca. 20 Sekunden die Absorption aufweist, welches dem *cis*/*trans -* Gleichgewicht des Substrates bei pH 7.6 entspricht, wurde ohne Zusatz von DnaK erhalten.
Die obere Kurve, die eine stärkere Krümmung als die untere Kurve aufweist, entspricht dem Datensatz, der durch eine Konzentration von 624 nM DnaK im Reaktionsansatz erhalten wurde. Durch die katalytische Wirkung von 624 nM DnaK stellt sich das Konformerengleichgewicht schon nach weniger als 10 Sekunden ein. Durch Nutzen der Auswertesoftware läßt sich bei Annahme eines Zeitgesetzes "Erster Ordnung" eine entprechende Geschwindigkeitskonstante (k_{obs}) berechnen. Für die unkatalysierte Reaktion ergibt sich eine Konstante von 0.36 s⁻¹ und für die katalysierte Reaktion eine Konstante von 0.55 s⁻¹. Die Abweichung der berechneten Meßpunkte der berechneten Kurve, durchgezogene Linie in Abb. 1, gegenüber den einzelnen gemessenen Werten, kann aus den unten aufgetragenen Werten (residuals versus time(s)) entnommen werden. Die statistische Schwankung der absoluten Abweichung über den Meßzeitraum belegt die Qualität der durchgeführten Nicht-linearen Regression zur Ermittelung der Geschwindigkeitskonstanten.

### Beispiel 2: Die APIase-Katalyse der cis/trans Isomerisierung einer Peptidbindung ist abhängig von der verwendeten APIase-Konzentration.

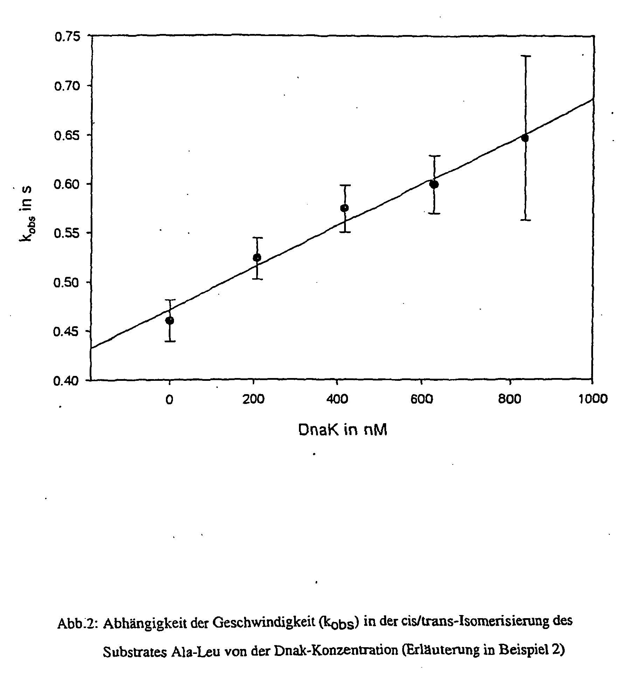

Ein wesentliches Kennzeichen chemischer Katalysatoren ist eine meßbare Dosis-Wirkungs-Beziehung. In Abhängigkeit von einer Änderung der Katalysatorkonzentration muß bei der Verwendung katalytischer Mengen eine solche Abhängigkeit zu beobachten sein. Eine solche Abhängigkeit ist in Abb. 2 bei der Katalyse der *cis*/*trans* Isomerisierungsgeschwindigkeit der Peptidbindung des Dipeptides Ala-Leu durch den Zusatz unterschiedlicher DnaK-Mengen zum Meßansatz dargestellt.

Die Messungen erfolgten mittels der in Beispiel 1 angegebenen Meßaparatur. Die Schichtdicke der verwendeten Quarzküvetten war 2mm, die Meßwellenlänge 220 nm. Folgende Lösungen wurden verwendet:
Pufferlösung: 12.5 mM Tris, 50 mM KCl, 11 mM MgCl₂.
Substratlösung: 20 mM Ala-Leu, in Aqua dest. bei pH 2.0 gelöst.
DnaK-Stammlösung: 20 µM in Pufferlösung a

Aus den Lösungen a und c wurden 4 Gebrauchslösungen d1,d2,d3 und d4 so hergestellt, daß nach Vermischen je einer Gebrauchslösung mit der Lösung c folgende DnaK-Konzentrationen in der Küvette erhalten wurden: 0, 208, 416, 625 bzw. 833 nM. Für das Substrat ergab sich durch diese Mischung immer eine Konzentration von 3.33 mM. Der pH der Lösung in der Küvette betrug jeweils 7.3. Die angegebenen Fehlerbalken ergeben sich aus jeweils drei unabhängigen Messungen für die Bestimmung der Geschwindigkeitskonstante k_{obs} bei einer DnaK-Konzentration.

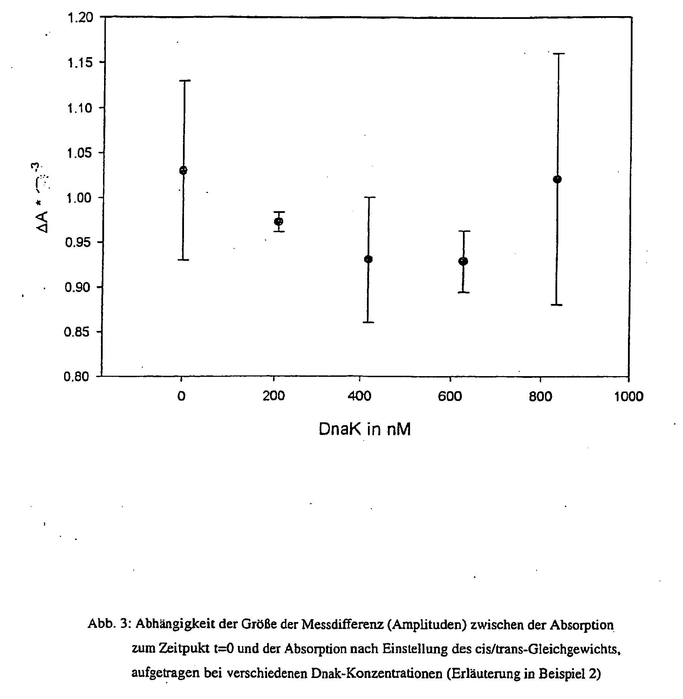

In Abb. 3 sind die sich aus den Messungen ergebenden Differenzen der Absorption, unmittelbar nach Start der Reaktion und der Absorption; die nach der Neueinstellung des *cis*/*trans* Isomerengleichgewichtes erhalten wird aufgetragen.

### Beispiel 3: Die Peptidyl-Prolyl-cisltrans Isomerase (PPIase) Cyp18 (EC 5.2.1.8; Accession number: P05092) oder das Referenzprotein Rinderserumalbumin besitzt keine nachweisbare APIase Aktivität:

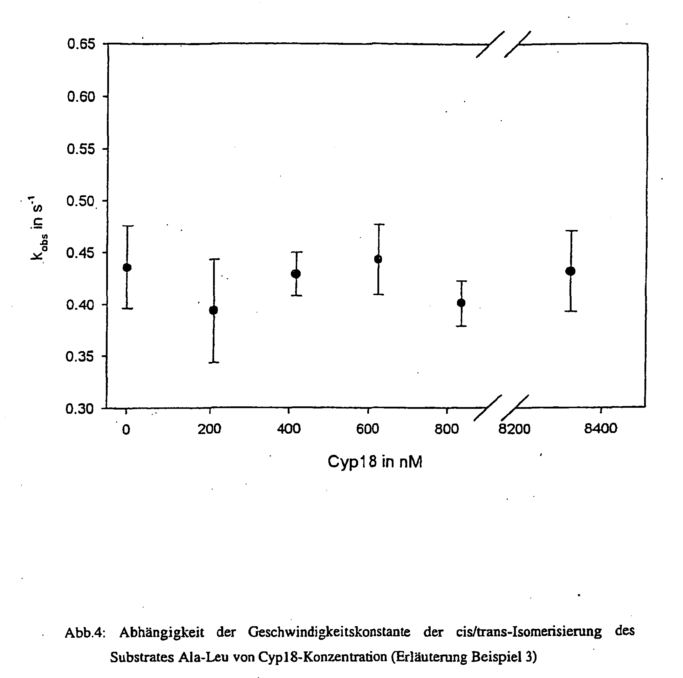

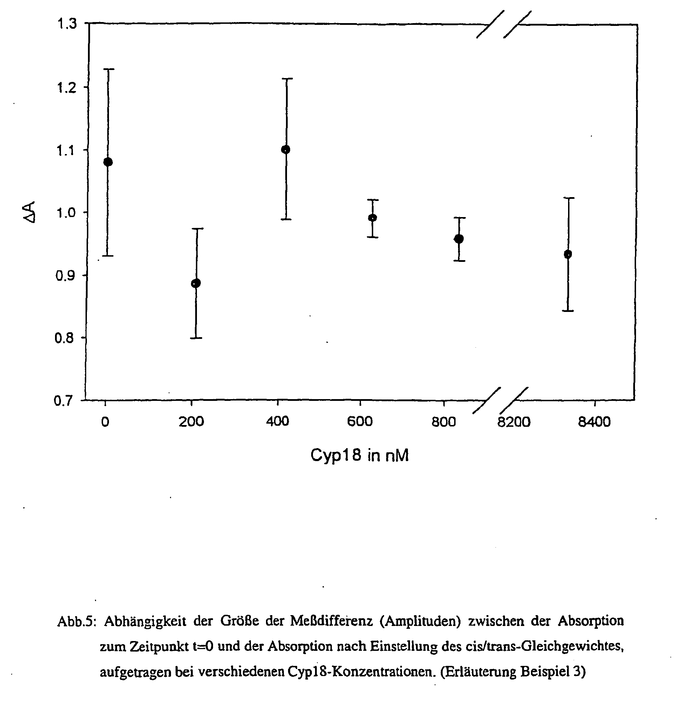

Die PPIase Cyp18, die zur PPlase-Familie der Cyclophiline gehört ist der Vertreter der PPIasen, der in Relation zu den unterschiedlichsten Mitgliedern von PPIase-Familien, phylogenetisch scheinbar am weitesten verbreitet ist (Maruyama T. and Furutani M., 2000, Frontiers in Bioscience 5:D821-D836). Die Substratspezifität dieses Cyclophilins gegenüber Peptidsubstraten von PPIasen überstreicht einen weiten Bereich. So katalysiert Cyp18 auch die *cis*/*trans*-Isomerisierung von phosphorylierten Ser-Pro bzw. Thr-Pro Peptidbindungen (Metzner M. et al J. Biol. Che. 276 :13524-13529 (2001); Schutkowski M. et al. Biochemistry 37:5566-5575 (1998)). Um zu untersuchen, ob Cyp18 die *cis*/*trans-*Isomerisierung des Dipeptides Ala-Leu katalysiert wurden in Gegenwart von bis zu 8.4 µM Cyp18 dessen Isomerisierungsgeschwindigkeit untersucht:
Humanes Cyp18 wurde entweder käuflich erworben (SIGMA-ALDRICH CHEMIE GmbH, Deutschland) oder als rekombinantes Enzym (E.coli-Kulturen) hergestellt. Die Cyclophilin-Konzentration der Cyclophilin-Stammlösung von 1.14 mM wurde mittels Titration gegen den Cyclophilin-Inhibitor Cyclosporin A (SIGMA-ALDRICH CHEMIE GmbH, Deutschland) ermittelt. Das verwendete Substrat Ala-Leu (BACHEM, Schweiz) wurde als 10 mM Stammlösung in Wasser mit einem pH von 2.0 hergestellt. Die verwendete Pufferlösung enthielt ein Gemisch folgender Chemikalien: 12 mM Tris, 50 mM KCl, 11 mM MgCl₂ und wurde mit HCl auf einen pH von 8.8 justiert. Die Messung der Isomerisierungsgeschwindigkeit des Substrates erfolgte am Stopped-Flow-Photometer (Applied Photophysics. Ltd, England) bei 25°C bei 228 nm unter Verwendung von 1 cm Quarz-Küvetten. Durch Zugabe des Substrates zur Mischung aus Puffer und Cyp18 wurden Lösungen in der Küvette mit Cyp18 - Konzentrationen von 0, 208, 416, 625, 833 und 8330 nM und jeweils 1.66 mM Ala-Leu mit einem pH von 7.9 hergestellt. Über einen Beobachtungszeitraum von 20 Sekunden, bei einer Auflösung < 0.05 Sekunden und einer Mischzeit < 0.1 Sekunde konnte keinerlei Beschleunigung der *cis* nach *trans* - Reaktion beobachtet werden (Abb. 4) Die ermittelten Geschwindigkeitskonstanten k_{obs} liegen unabhängig von der verwendeten Cyp18-Konzentration in der Größenordnung von etwa 0.43 ±0.03 s⁻¹.
Statt Cyp18 wurde in einem weiteren Experiment gereinigtes Rinderserumalbumin (SIGMA-ALDRICH CHEMIE GmbH, Deutschland) in einer Konzentration von 1µM eingesetzt. Zur Reinigung des Rinderserumalbumins wurden Ionenaustausch- und Affinitätsmaterialien verwendet. Rinderserumalbumin vermochte ebenso wie Cyp18 die *cis*/*trans* Isomerisierung des Dipeptides nicht zu katalysieren.
Ein Maß der Qualität der erhaltenen Messwerte sind die Amplituden der Messsignale, d.h. die Differenz der gemessenen Absorption unmittelbar nach Zusammenmischen aller Lösungen und der gemessenen Absorption nach Ablauf der beobachteten Reaktion. Die nichtkatalysierte *cis*/*trans*-Isomerisierung des Substrates Ala-Leu erreicht unter den gewählten Messbedingungen innerhalb 20 Sekunden das Gleichgewicht.
Die Absorptionsdifferenz der Amplituden beträgt etwa 1.0±0.2 x10⁻³. Wie Abb. 5 zeigt, sind die erhaltenen Absorptionsdifferenzen, der in Abb. 4 zusammengefaßten Messungen, unabhängig von der Typ18 Konzentration.

### Beispiel 4: Das Protein DnaK besitzt APIase, aber keine PPIase-Aktivität.

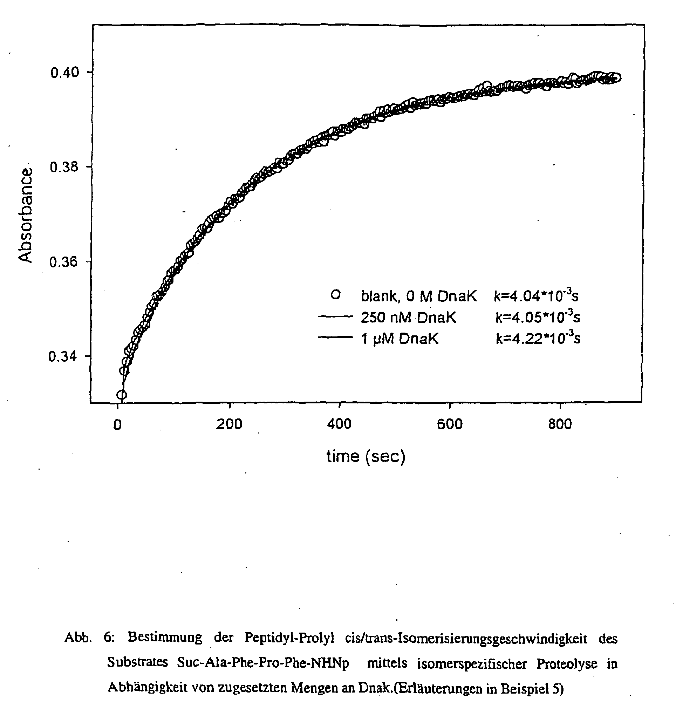

Wie Beispiel 3 zeigt, ist es nicht möglich mittels katalytischer Mengen der PPIase Cyclophilin, die *cis*/*trans* Isomerisierung einer Peptidbindung in einem Dipeptid, zusammengesetzt aus Aminosäuren, zu katalysieren. Erfindungsgemäß gelingt eine solche Katalyse mit Substanzen, welche APIase-Aktivität besitzen. Wie im weiteren gezeigt wird, ist die APIase-Aktivität der APIase DnaK spezifisch und schließt die Katalyse der *cis*/*trans -* Isomerisierung von Prolyl-Peptidbindungen aus.
Zum Nachweis einer PPIase-Katalyse existieren zahlreiche Verfahren (z.B.: Fischer, G. (1994), Angew. Chemie Intl.ed. Engl. 33, 1415-1436). Zu den am meisten angewendeten Nachweismethoden gehört die isomerspezifische Hydrolyse. Diese Methode nutzt die Regio-Spezifität verschiedener Proteasen, wie Chymotrypsin oder Subtilisin gegenüber in P2'-Position zu Prolylpeptidbindung stehenden Proteasespaltstellen aus (Fischer, G., Bang, H. & Mech, C., 1984, Biomed. Biochim. Acta, 43:1101-1111).
Die Details des Beispiels 5 werden im folgenden beschrieben:
Messgerät: Spektralphotometer Diodenarray HP8452A (Hewlett Packard, USA)
Substrat: Suc-Ala-Phe-Pro-Phe-NHNp (BACHEM, Schweiz)
Protease: Chymotrypsin (Merck, Deutschland)
Messtemperatur: 10°C
Puffer: 35 mM Hepes, pH 7.8
Proteasegebrauchslösung: 5 mg Protease je ml Puffer
Substratgebrauchslösung: 35 mg Substrat gelöst in 1 ml DMSO
DnaK-Gebrauchslösung: 30µm in Puffer
Küvette: 1ml Quarzküvette, Schichtdicke 1 cm
Messwellenlänge: 390 nm
Ablauf: Nach Temperieren eines Gemischs aus Substrat, DnaK und Puffer, wird die Reaktion durch schnelle Zugabe und homogener Verteilung der Proteasegebrauchslösung gestartet. In der Küvette wird dadurch eine Chymotrypsinkonzentrationen von 0.83 mg/ml erreicht. Durch die in der Küvette enthaltene Substratmenge wird nach vollständigem Umsatz des Substrates eine Endextinktion von ca 0.43 erreicht. Die in der Küvette enthaltenen Konzentrationen an DnaK lagen bei 0, 250 und 1000 nM.
Ergebnis: DnaK zeigte keinen sichtbaren Einfluß auf die *cis*/*trans* Isomerisierungsgeschwindigkeit des Substrates. Die berechneten Geschwindigkeitskonstanten k_{obs} der cis/trans-Isomerisierung des Substrates zeigten im Fehlerbereich keine Abweichung zur Geschwindigkeitskonstante, die ohne DnaK (blank) erhalten wurde. Abb. 6 zeigt die übereinandergelegten Meßdaten der drei Meßserien. Dabei entsprechen die Kreise der Messung ohne DnaK und die Punkte den Messungen mit DnaK-Zusatz.

### Beispiel 5: Screening von Peptidbibliotheken

Da die Bindung von Molekülen an ein entsprechendes Targetenzym einen ersten Hinweis auf die Möglichkeit des Auffindens eines Inhibitors ergibt, und Peptidbibliotheken es ermöglichen solche potentiellen Inhibitoren zu finden wurde eine entsprechende Bibliothek ( Z. Songyang et al., 1993, Cell 72:767) verwendet. Bei Verwendung aller natürlichen Aminosäuren mit der Ausnahme von Cystein, bei Verwendung äquimolarer Mengen in jeder degenerierten Position, werden theoretisch für zwei Bibliotheken 19⁶ = 4.7x10⁷ unterschiedliche Peptidsequenzen erzeugt. Zum Kuppeln der APIase DnaK wurde als Matrix eine aktivierte Agarose (Aff-Gel 10, BIO-RAD Laboratories München) entsprechend den Vorschriften des Herstellers benutzt. Nach Inkubation der aus der Bibliothek erhaltenen Peptidmischung mit der DnaK-Sepharose und extensivem Waschen des DnaK-Gels mit 1 mM Ammoniumazetat-Puffer (pH 7.4) können gebundene Peptide mit 30%-iger Essigsäure eluiert und sequenziert werden. Die aufgefundenen, an DnaK bindende Peptidsequenzen können konventionell in mg-Mengen synthetisiert und mittels Massenspektrometrie und NMR validiert validiert werden. Zur Bewertung der Inhibierung eines solchen Peptids gegenüber der APIase-Aktivität von DnaK muß das Peptid dem in Beipiel 1 beschriebenen Aktivitätsassay zugesetzt werden. Die Konzentration an Peptid, welche ausreicht, um die APIase-Aktivität um 50% zu inhibieren (IC₅₀-Wert) wird genutzt um die Inhibitionskraft einzelner Inhibitoren zu vergleichen.

### Beispiel 6: Die APIase-Aktivität von DnaK lässt sich durch natürlich vorkommende Peptide inhibieren.

Die erhaltenen IC₅₀-Werten (Beispiel 5) von Peptidsequenzen, welche die APIase-Aktivität inhibieren, lassen sich mit natürlichen Peptidsequenzen vergleichen, über die in der wissenschaftlichen Literatur bereits Kenntnisse vorhanden sind. Diese Kenntnisse sind z.B. mittels Literatursuche in Literaturdatenbanken (z.B.: MEDLINE, CURRENT CONTENTS) aber auch durch Verfolgen der Fachliteratur oder Patentbibliotheken (z.B.: DELPHION) dem Fachmann zugänglich. So weisen z.B. einzelne der mit Peptidbibliotheken erhaltenen und durch APIase-Aktivitätstests verifizierten Daten darauf hin, dass die im Peptidhormon Substanz P (z.B. Jessell TM., 1982, Nature 295:551) vorkommende Peptidsequenz (-Arg-Pro-Lys-Pro-Gln-Gln-Phe-Phe-Gly-Leu-Met-) geeignet sein sollte, die APIase-Aktivität von DnaK zu inhibieren.
Um dies zu testen, wurde Substanz P (BACHEM Biochemica GmbH, Heidelberg) in steigenden Konzentrationen 1 bis 100 µM dem spektrophotometrtischen Aktivitätstest der APIase-Aktivität von DnaK (Beispiel 1) unterzogen. Aus der Dosis-Wirkungs-Kurve (Abb.
7), der Hemmung der APIase-Aktivität durch Substanz P kann ein IC50-Wert von 76 ± 16 µM erhalten werden.

### Beispiel 7: In Homogenaten biologischer Proben existieren Proteine mit APIase-Aktivität.

Um mögliche Proteine mit APIase-Aktivität aufzufinden wurden biologische Proben des Mikroorgansimus Escherichia coli (E. coli) und eines Säugetiers (Haus-Schwein) genutzt. E.coli-Homogenat: Die Bakterien wurden nach Überführung in eine Suspensionskultur entsprechend der Standardvorschrift bei 30 °C im Schüttelkolben gehalten und die Zunahme der Zellmasse durch spektroskopische Messung der Zelldichte verfolgt. Unmittelbar nach Erreichen der logarithmischen Wachstumsphase der Bakterien wurden die Zellen durch Zentrifugieren geerntet. Nach 2-maligen Waschen der Zellen mit Waschpuffer, wurden die Zellen mittels Druckabfall (French-Press) zerstört. Durch hochtouriges Zentrifugieren bei 80 000 g für 10 Minuten wurde ein klarer Überstand erhalten.
Homogenat von Schweinehirn: Von einem Schweinehirn, welches unmittelbar nach der Entnahme mittels flüssigem Stickstoff tiefgefroren war, wurden 10 g in kleinen Stücken abgeschnitten und mit 1 ml Lysispuffer im Eisbad versetzt. Nach dem schonenden Zerkleinern der Probe mittels Scherkräften (Elvejheim-Potter, Niederlande) und Zentrifugation bei 80 000 g für 20 Minuten konnte nach Verwerfen eines oberen inhomogenen Überstandes eine mittlere klare Fraktion erhalten werden.

Sowohl der aus Batterien erhaltenen Überstand (A) als auch die aus Schweinehirn erhaltene Fraktion (B) wurden dem im Beispiel 1 beschriebenen Äktivitätstest unterworfen. Verdünnungen beider Überstände konnten die *cis*/*trans*-Isomerisierung des Substrates Ala-Leu merklich beschleunigen. Erhitzung beider Überstände für 30 Minuten auf 70 °C führte zur vollständigen Inaktivierung der beschleunigenden Aktivität.
Durch Anwenden mehrfacher Reinigungsschritte und unter Hinzuziehung der MALDI-Massenspektrometrie konnte aus einer aktiven Fraktion des E. coli - Überstandes (A) das in Beispiel 1 aufgeführte DnaK als Protein identifiziert werden, welches APIase-Aktivität aufweist.

### Beispiel 8: Nachweis der cis/trans Isomerisierung einer einzelnen Peptidbindung eines Pentapeptides und der Katalyse dieser Isomerisierung durch die APIase-Aktivität des Proteins DnaK mittels NMR Messungen.

Neben der Bestimmung der *cis*/*trans -* Isomerisierung sekundärer-Amid Peptidbindungen mittels UV-VIS Messungen, wie in vorherigen Beispielen aufgeführt, ist die Isomerisierung dieser Peptidbindung, wie zahlreiche Veröffentlichungen belegen, auch mit anderen Methoden meßbar. So konnte z.B. die Geschwindigkeit der *cis*/*trans*-Isomeriserung des Dipeptides Gly-Gly an Hand des Raman Spektrums bei 206 nm bestimmt werden (Li P. et al. JACS (1997) 119:1116-11120). Bei genauer Kenntnis der dreidimensionalen Struktur und dem Vorhandensein besonderer spektroskopischer Eigenschaften gelingt es auch bei Proteinen oder Oligopeptiden die *cis*/*trans* Isomerisierungsgeschwindigkeit einzelner sekundärer-Amid Peptidbindungen mittels spektroskopischer Methoden zu ermitteln. Gut untersucht ist so die Bestimmung der *cis*/*trans -* Iosmerisierungsgeschwindigkeit der Tyr38-Ala39 Bindung in dem Protein Rnase T₁ (Odefey C. et al., 1995, J.Mol.Biol. 245:69-78; Mayr L. et al., 1994, J.Mol.Biol. (1994) 240:288-293; Dodge RW. et al., 1996, Biochemistry 35:1548-1559).
Eine weitere, erst 1998 beschriebene Möglichkeit, ist die Bestimmung der *cis*/*trans-*Isomerisierungsgeschwindigkeit sekundärer-Amid Peptidbindungen die aromatische Aminosäuren enthalten, mittels 2D 1H NMR-Austauschexperimente (Scherer G. et al., 1998, JACS 120:5568-5574). In Beispiel 8 wird diese NMR-Methodik genutzt, um eine katalytische Beschleunigung der *cis*/*trans*-Isomerisierung der Peptidbindungen Ala-Tyr und Tyr-Ala innerhalb des Pentapeptides Ala-Ala-Tyr-Ala-Ala nachzuweisen. Die Beschleunigung wird durch katalytische Mengen an DnaK erreicht.

### Material und Methode:

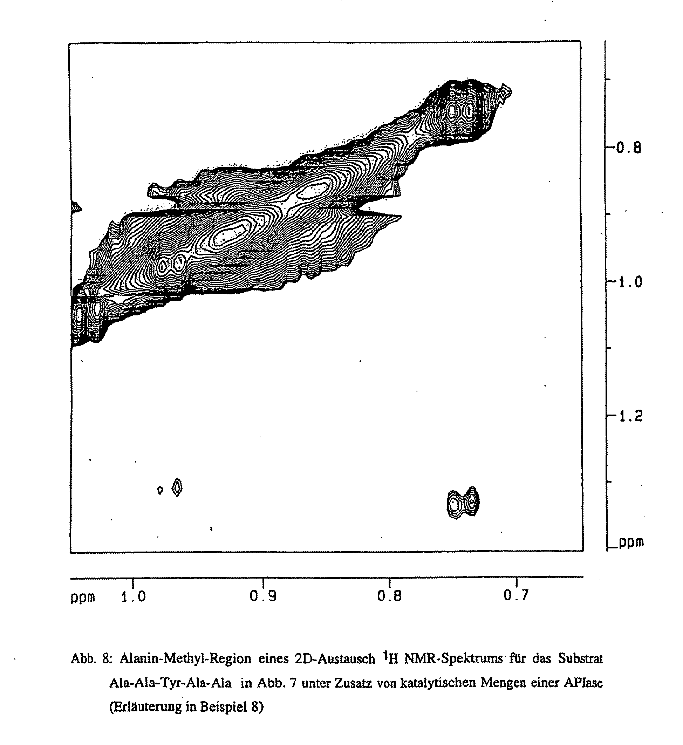

NMR-Gerät: Bruker DRX-500, ausgerüstet mit z-Gradient
Abb. 8: Der Alanin-Methylbereich aus dem zweidimensionalen Austausch 1H-NMR-Spektrum von 25 mM Ala-Ala-Tyr-Ala-Ala in 25 mM Tris, 11 mM MgCl₂, 50 mM KCl, 9:1 H₂O:D₂O, pH7.1 bei 278 °K. Die Mischzeit des phasensensitiven NOESY betrug 330 ms. Jeder FID bestand aus 184 *scans.* Die Datenmenge aus t₁ x t₂ = 512 x 8192 Punkten erstreckte sich über eine spektrale Breite von 5501 hz x 5482 Hz. Die Basislinienkorrektur wurde durch eine Anpassung mit einem Polynom fünfter Ordnung in der F₂-Dimension durchgeführt. Die Intensitäten des Spektrums, d.h. die Niveaus in der zweidimensionalen Darstellung, wurden auf ein von der Peptidstruktur unabhängiges Kreuzsignal vergleichbarer Intensität normiert, nämlich das vom chemischen Austausch zwischen den beiden ¹³C-Satelliten von ¹Ala hervorgerufene Kreuzsignal.

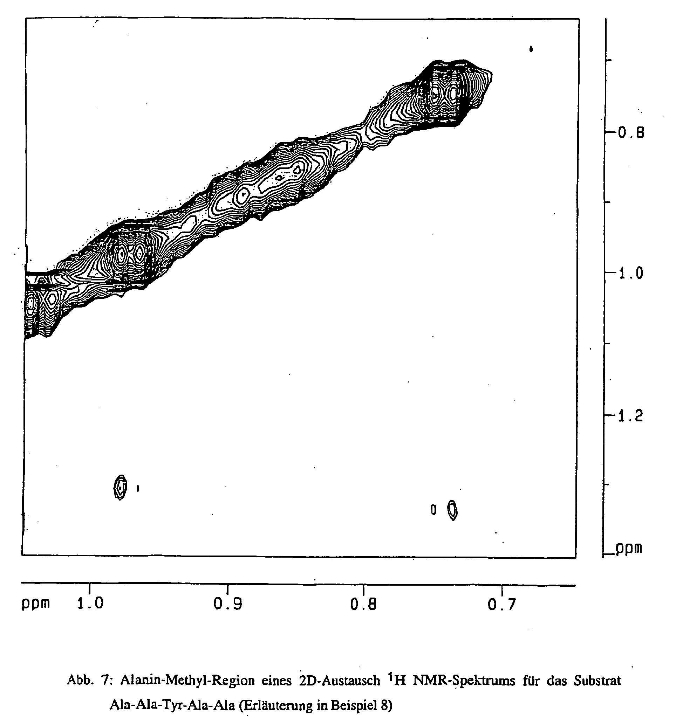

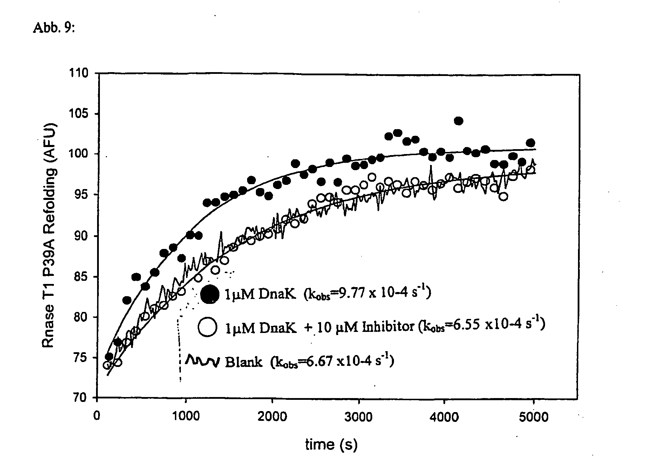

Abb. 9: Gleicher Ausschnitt aus dem zweidimensionalen Austausch 1H-NMR-Spektrum von 25 mM Ala-Ala-Tyr-Ala-Ala in 25 mM Tris, 11 mM MgCl₂, 50 mM KCl, 9:1 H₂O:D₂O, pH 7.1 bei 278 °K wie in Abb. 7. Das Experiment wurde unter identischen Bedingungen wie oben durchgeführt. In der Meßlösung war zusätzlich 20 µM DnaK enthalten.
Ergebnis: Die aromatische Aminosäure Tyrosin flankieren im Pentapeptid zwei Alanine. Die der cis-Konformation entsprechenden CH₃-Signale der zwei Alanine lassen sich im zweidimensionalen NOESY-Experiment an Hand der "Cross Peaks" bei 0.74 ppm und 0.97 ppm lokalisieren. Die zugehörigen Signale für die trans-Konformation sind bei 1.33 ppm bzw.
1.31 ppm zu finden (in den Abbildungen nicht gezeigt). Die chemischen Verschiebungen entsprechen den Verschiebungen, die für die CH₃-Alanin-Signale von Tyr-Ala (0.33 ppm) und Ala-Tyr (0.57 ppm) bestimmt wurden (Scherer et al. 1998). Aus der Form der ¹H NMR Signale und ihrer sensitiven Veränderung bei Änderung der Isomerisierungsgeschwindigkeit lassen sich die *cis*/*trans*-Isomerisierungsgeschwindigkeiten mittels mathematischer Methoden berechnen. Eine Verbreiterung der ¹H NMR Signale entspricht dabei der Zunahme der Isomerisierungsgeschwindigkeit. Wie Abb. 8 zeigt, verbreitert sich, beim Vergleich beider Kreuzsignale, unter dem Einfluß katalytischer Mengen an DnaK hauptsächlich das Kreuzsignal, welches der Ala-Tyr - Peptidbindung entspricht. Dies bedeutet, daß die APIase-Aktivität des DnaK eine ausgeprägte Substratspezifität aufweist. Im Pentapeptidsubstrat Ala-Ala-Tyr-Ala-Ala wird die *cis*/*trans* - Isomerisierung der Ala-Tyr - Peptidbindung besser katalysiert als die der Tyr-Ala - Peptidbindung.

### Beispiel 9: Quantifizierung der APIase-Aktivitätsmessung mittels NMR

In weiterer Auswertung der in Beispiel 8 aufgeführten Methodik des zweidimensionalen NOESY-Experimentes lassen sich die Kreuzsignale mittels üblicher mathematischer Techniken quantifizieren. Durch die Nutzung relativer Intensitäten, bezogen auf ein von der Peptidstruktur unabhängiges Kreuzsignal vergleichbarer Intensität (Kreuzsignal, welches durch chemischen Austausch zwischen den beiden ¹³C-Satelliten von ¹Ala hervorgerufen wird) kann der Einfluß verschiedenster Zusätze auf die Geschwindigkeit der *cis*/*trans* Isomerisierung der Peptidbindungen Ala-Tyr und Tyr-Ala im Pentapeptid Ala-Ala-Tyr-Ala-Ala bestimmt werden (Tab. 1)

**Tab. 1 Relative Intensitäten der Kreuzsignale der Peptidbindungen Ala-Tyr und Tyr-Ala des Pentapeptides Ala-Ala-Tyr-Ala-Ala in Abhängigkeit von Zusätzen. Bedingungen wie in Beispiel 8 angegeben.**

| | Ala-Tyr | Tyr-Ala |
|---|---|---|
| Kein Zusatz | 3.50 ± 0.51 | 3.89 ± 0.32 |
| 20 µM DnaK*) | 6.37 ± 0 . 5 7 | 3. 3 9 ± 0.4 5 |
| 20 µM FKBP12*) | 3.94 ± 0.87 | 2.68 ± 0.75 |
| 20 µM Parvulin*) | 2.90 ± 0.96 | 3.11 ± 0.46 |
| 20 µM Trigger-Faktor (TF) *) | 3.83 ± 1.07 | 4.42 ± 0.58 |
| 20 µM Fragment des TF*) | 3.96 ± 0.77 | 3.22 ± 0.58 |

| | | |
|---|---|---|
| *)Alle Zusätze wurden rekombinant hergestellt, bzw. wurden käuflich erworben. Humanes DnaK und FKBP12 wurden erhalten von SIGMA-ALDRICH GmbH (Deutschland), Parvulin (Rahfeld et al., 1994, FEBS Letters 352:180-184), Trigger-Faktor (Stoller G. et al., 1995, EMBO J. 14:4939-4948) und ein Fragment des Trigger-Faktors (Stoller G. et al., 1996, FEBS Letters 384:117-122) waren Aliquote der in den zitierten Arbeiten hergestellten Proteine, bzw. wurden analog hergestellt. | | |

### Bewertung:

Die APIase-Aktivität der APIase DnaK ist regiospezifisch. Während die *cis*/*trans -* Isomerisierung der Ala-Tyr - Peptidbindung durch 20 µM DnaK so beschleunigt wird, daß sich die relative Intensität des Kreuzsignals fast verdoppelt, hat diese Konzentration an DnaK unter den gewählten Bedingungen keinen Einfluß auf die *cis*/*trans-*Isomerisierungsgeschwindigkeit der Tyr-Ala - Peptidbindung.
Die Peptidyl-Prolyl *cis*/*trans* Isomerasen FKBP12, Parvulin und Trigger-Faktor, bzw.
Trigger-Faktor - Fragment katalysieren weder die die *cis*/*trans -* Isomerisierung der Ala-Tyr - Peptidbindung noch die die *cis*/*trans -* Isomerisierung der Tyr-Ala - Peptidbindung des Pentapeptides Ala-Ala-Tyr-Ala-Ala.

### Beipiel 10: APIase-Inhibitoren haben antibakterielle Eigenschften.

Wie in Beispiel 6 angegeben, ermöglichen die durch Peptidbanken und mittels APIase-Aktivitätstest erhaltenen Informationen natürliche, d.h. bereits bekannte APIase-Inhibitoren aufzufinden. Eine dieser potentiellen natürlichen APIase-Inhibitoren ist die Sequenz VDKGSYLPRPTPPRPIYNRN, die als antibakterielles Peptid Pyrrhocoricin aus Insekten (Otvos L., 2000, J Peptide Science 6:497-511) allerdings als am Threonin glykosyliertes Peptid isoliert wurde. Pyrrhocoricin kann nach Standardmethoden (Festphasensynthese, Fields GB. Et al., 1990, Int J Pept. Protein Res 35:161-214) hergestellt werden. Die Reinigung des Peptides gelingt mittels RHPLC ("Reversed-phase high-performance liquid chromatography), die Validierung des Peptides mittels Massenspektrometrie. Die Peptidmenge des lyophilisierten Produktes kann mit Hilfe der UV-VIS Spektroskopie an hand der Peptidbindung bestimmt werden. Mit dem in Beispiel 1 angegebenen APIase-Aktivitätsassay kann die Inhibition der APIase-Aktivität von DnaK durch das Peptid Pyrrhocoricin bestimmt werden. Obwohl das Peptid im Unterschied zur aus Insekten isolierten Verbindung nicht glykolysiert ist, hemmt es die APIase-Aktivität des DnaK.
Die antibakterielle Aktivität des APIase-Inhibitors kann wie bei Hoffmann et al. (1999, Biochim. Biophys. Acta 1426:459-467) beschrieben, gegenüber kultivierten Bakterien vorgenommen werden. Gegenüber dem Escherichia coli Stamm D22 kann für diese Substanz einen IC₅₀-Wert von 150 nM bestimmt werden.

### Beispiel 11: Messung der Wirkung von APIase-Inhibitoren auf die krankmachende Faltung von Prionen-Protein.

Der Unterschied zwischen natürlichen Prionenprotein PrP^{C} und dem "falsch" gefalteten krankmachenden oder die Krankheit kennzeichnenden Scrapie-Prionenprotein PrP^{Sc} kann mittels kommerzieller Assays, so z.B. an Hand der unterschiedlichen Hydrolyse-Stabilität beider Proteine (z.B. Bueler H. et al., 1994, Mol. Med. 1:19-30) bestimmt werden.
Die Umwandlung von PrP^{c} in PrP^{Sc} gelingt
a) durch Inkubation einer 1000-fachen Verdünnung von Hirnhomogenaten von Hamstern, die mit Scrapie infiziert sind, d.h. bei denen sich eindeutig und in großer Menge PrP^{Sc}-Proteine in Hirnhomogenaten feststellen lassen, mit
b) 5% Hirnhomogenat von gesunden Hamstern und
c) durch Einwirken von Ultraschall, wie bei Saborio GP. et al., (2001, Nature 411:810-813) beschrieben.
Nach 10 bis 20 Ultraschallzyklen kann die Zunahme von PrP^{Sc} im Inkubationsansatz eindeutig als Unterschied zur Kontrolle (Inkubationszyklen ohne Ultraschall) im Immunoblot nachgewiesen werden. Durch Zusatz eines oder eines Gemisches von APIase-Inhibitoren zu einem solchen PrP^{C}-PrP^{Sc}-Umwandlungsansatz, kann deren Wirkung als Wirkstoff zur Inhibition der Umwandlung von PrP^{C} in PrP^{Sc} quantifiziert werden. Solche Wirkstoffe sind z.B. zyklische Tetrapyrrole (wie Porphyrine und Phthalocyanine; Priola SA. Et al., 2000, Science, 287:1503-1506) aber auch Oligopeptide wie z.B. die vom Prion abgeleitete Sequenz AAAAGAVVGGLGGYMLGSAMSRPMMHV (Chabry J. et al. 1998, J. Biol. Chem. 273:13 203-13 207).

### Beispiel 12: APIase katalysiert die Rückfaltung eines Proteins mit einer cis-Peptidbindung

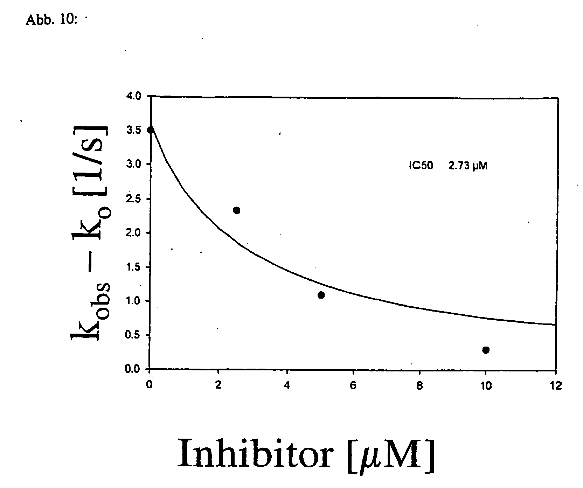

RNAse T1 enthält an der Sequenzstelle 38/39 eine Ser-Pro Sequenz mit einer cis-Konformation. Rückfaltungsexperimente mitRNAse T1 zeigen, dass die Rückfaltungsgeschwindigkeit von der Konformation dieser Peptidsequenz beeinflusst wird. Im Detail wird dazu das protein durch geeignete Zusätze entfaltet und die Renaturierungsgeschwindigkeit nach Entfernen dieser Zusätze, z.b. durch schnelles verdünnen, registierend gemsessen. Wie sich zeigt (Scholz et al. Biol. Chem 1998, March; 379(3): 361-5) führt der Austausch des Prolin 39 durch ein Alanin (P39A) zu einer Proteinmutante, deren Rückfaltungsgeschwindigkeit weiterhin von dieser Peptidbindung beeinflußt wird. Zugegsetzte APIase katalysiert konzentrationsabhängig die Isomerisierung dieser Nicht-Prolylpeptidbindung und somit die Rückhaltung der P39A Mutente. Ein solches Rückfaltungsexperiment wie in Scholz et al. Biol Chem 1998, 379(3): 361-365 beschrieben zeigt Abb. 9. Während ohne APIase-Zusatz die Rüchfaltungsgeschwindigkeit eine Geschwindigkeitskonstante von 6,67 x 10^{-4-s} aufweist, wird mit Zusatz von 1µM Dank eine Beschleunigung auf 9,77 x 10^{-4-s} beobachtet. Der Zusatz von 10 µM Inhibitor 37/B10 verursacht eine solche Inhibierung, dass die Rückfaltungsgeschwindigkeit der Geschwindigkeit entspricht, die ohne Inhibitor gemessen wurde. Aus der Auftragung der gemessenen Geschwindigkeiten der Rückfaltung bei verschiedenen Inhibitorkonzentrationen, kann wie in Abb. 10 gezeigt die Effektivität des Inhibitors z.B. als IC50 bestimmt werden.

### Assayansatz:

28mM Rnase T1 P39A wurde bei 15°C für 60 min mit 5 M Guanidinium-Cl und 0.1M Tris-HCl bei pH 8 inkubiert. Die Rückfaltung bei 15°C wurde durch 40-fache Verdünnung des entfalteten Proteins in Rückfaltungspuffer (0.1 Tris-HCl, 50mM KCl, 11mM MgCl₂) bei pH 8 gestartet. Die Rückfaltung wurde als Zunahme der Proteinfluoreszenz bei 320 nm (10 nm Bandweite) bei einer Anregung von 278 nm (1,5 nm Bandweite) mit einem Jobin-Yvon-Spex Fluoromax -2 Fluoreszenzspektrometer bestimmt.

Auf die selbe Weise wurden, wie in untenstehender Tabelle noch weitere IC50-Werte bestimmt.

| Substanz | *IC50 [µM]* |
|---|---|
| 37B10 | 2.73 |
| 37/B11 | 5.9 |
| 30/B4 | 7.5 |
| 53/B5 | 26.4 |
| 28/C8 | 55.3 |
| 28/C3 | 74.6 |
| 28/B7 | 177.1 |

## Patentansprüche

1. Arzneimittel umfassend ein Peptid oder Peptidmimetikum mit der allgemeinen Grundstruktur
Y-R₂-R₃
wobei Y eine gesättigte oder ungesättigte, gerad- oder verzweigkettige Fettsäure ist, die mit der Aminosäure oder dem Aminosäuremetikum R₂ über eine C(O)NH-Bindung verknüpft ist, und wobei die Aminosäure oder das Aminosäuremetikum R₃ über seine Aminogruppe mit R₂ über eine C(Q)NH-Bindung verknüpft ist, wobei R₂ alle natürlichen Aminosäuren oder ihre Mimetika und R₃ die Aminosäuren Methionin, Alanin, Serin, Glutaminsäure, Leucin, Lysin, Isoleucin und/oder Glycin oder ihre Mimetika umfasst und gegebenenfalls einen pharmazeutisch verträglichen Träger.

2. Arzneimittel nach Anspruch 1, wobei das Peptidmimetikum R₃ aus mindestens 5 und maximal 29 Atomen besteht und fähig sein muss, eine solche chemische Bindung mit R₂ eingehen zu können, die der Struktur einer Peptidbindung entspricht, wobei die Grundstruktur von R₃ mit H₂N-R-Z beschrieben wird, wobei Z für eine funktionelle Gruppe ausgewählt aus Nitro-, Sulfoxy, Phospho-, Amino, Carboxy, Sulfhydryl steht, wobei R für eine geradkettige oder verzweigte Kohlenwasserstoffkette steht, deren maximale Länge 14 Kohlenstoffatome und minimal 1 Kohlenstoffatom enthält, wobei der Abstand zwischen H₂N- -Z über R maximal 4 Kohlenstoffatome und minimal 1 Kohlenstoffatom beträgt, wobei R selbst gegebenenfalls mit einer oder bis zu drei Hydroxyl-, Carbonsäure-, Carbonsäureester-, Amid-, Aldehyd-, Ether-, Iminoether-, Hydrazid-, Izidoether-, Thiol- oder Sulfoximgruppen versehen sein kann.

3. Arzneimittel nach Anspruch 1 oder 2, wobei die Fettsäure 8 bis 24 Kohlenstoffatome aufweist.

4. Arzneimittel nach einem der Ansprüche 1 bis 3, wobei die Fettsäure 14 bis 16 Kohlenstoffatome aufweist.

5. Arzneimittel nach einem der Ansprüche 1 bis 4, wobei die Fettsäure einfach hydroxyliert ist.

6. Arzneimittel nach einem der Ansprüche 1 bis 4, wobei die Fettsäure mehrfach hydroxyliert ist.

7. Arzneimittel nach einem der Ansprüche 1 bis 6, wobei die Aminosäuremimetika R₂ und R₃ gleich oder verschieden sind und bis zu 34 Kohlenstoffatome haben und eine lineare Struktur, die gegebenenfalls verzweigt sein kann, ein C5- bis C34-carbozyklische Struktur oder eine heterozyklische Struktur, enthaltend S, N, und O als Heteroatome und Ringgrößen von 5 bis 8 haben, wobei diese Strukturen vollkommen gesättigt sein können oder über ein- oder mehrfache ungesättigte Kohlenstoffgruppierungen verfügen können.

8. Arzneimittel nach Anspruch 7, wobei R₂ und R₃ benzoine oder nichtbenzoide Aromaten einschließen, die gegebenenfalls ein- oder mehrfach mit funktionellen Gruppen ausgewählt aus Nitro-, Sulfoxy-, Phospho-, Amino-, Carboxy-, Sulfhydrylgruppen substituiert sein können.

9. Arzneimittel nach Anspruch 7 oder 8, wobei R₂ und R₃ ein- oder mehrfach halogeniert sind, wobei Halogen ausgewählt ist aus Fluor, Chlor, Brom oder Jod.

10. Arzneimittel nach Anspruch 1 bis 9, wobei Y eine gesättigte Fettsäure ist.

11. Arzneimittel nach Anspruch 1 bis 9, wobei Y eine ungesättigte Fettsäure ist.

12. Arzneimittel nach Anspruch 10, wobei die gesättigte Fettsäure ausgewählt ist aus: propionsäure, Buttersäure, iso-Buttersäure, Valeriansäure, iso-Valeriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure. Nonadecansäure, iso-Tuberculostearinsäure, Arachinsäure, Behensäure, Lignocerinsäure, Cerotinsäure und Melissinsäure.

13. Arzneimittel nach Anspruch 12, wobei die ungesättigte Fettsäure ausgewählt ist ans: Acrylsäure, Crotonsäure, Palmitoleinsäure, Ölsäure, Erucasäure, Sorbinsäure, Elaeostearinsäure, Arachidonsäure, Clupanodonsäure, Docosahexaensäure, Elaidinsäure, Linolsäure und Linolensäure.

14. Arzneimittel nach einem der Ansprüche 1 bis 13, wobei R₂ und R₃ zusammen ein Peptidmimetikum darstellen.

15. Arzneimittel nach Anspruch 1 bis 14 umfassend eine Verbindung mit der Formel: 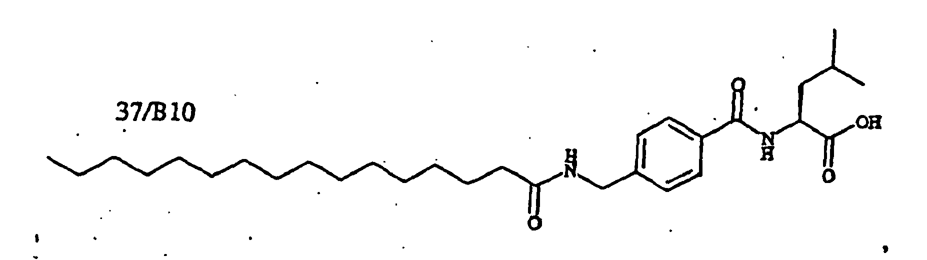 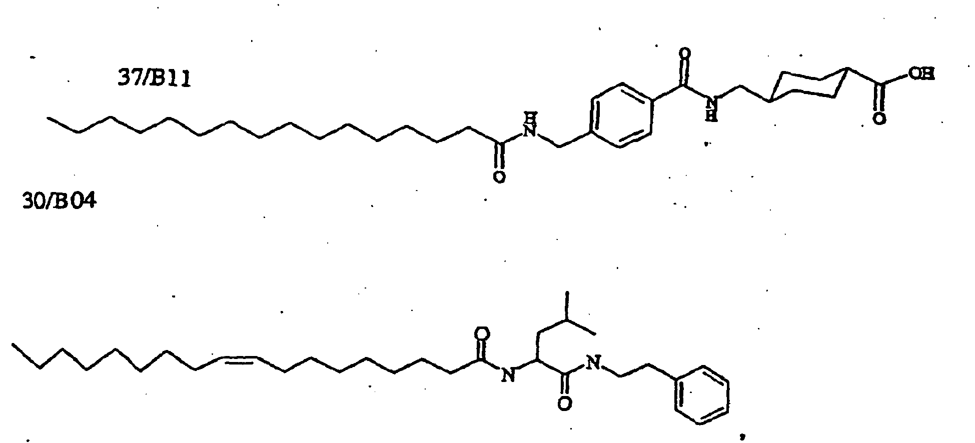 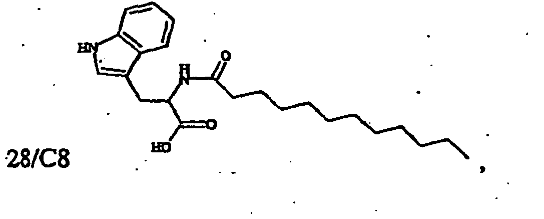 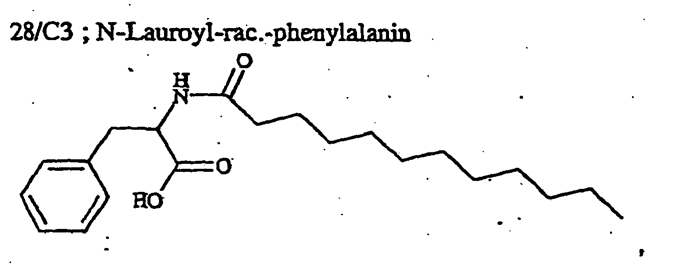 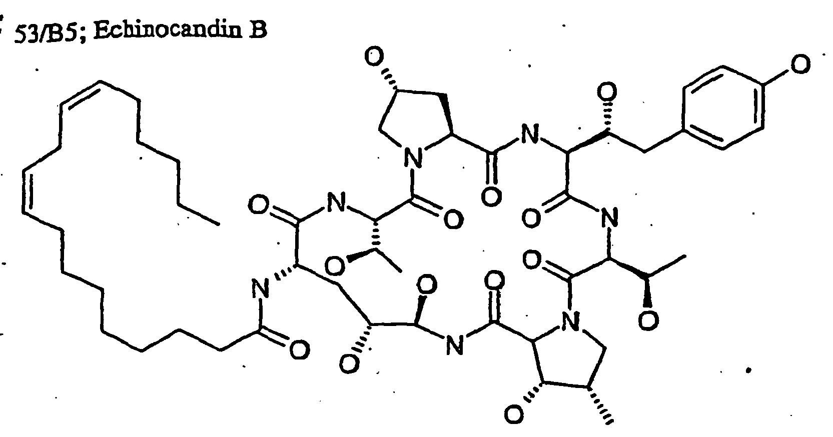 und

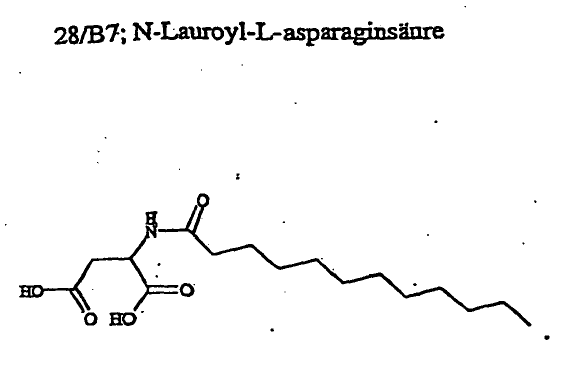

16. Eine in vitro Methode um Zellwachstum durch Inhibierung einer APIase-Aktivität zu inhibieren, in dem die entsprechenden Zellen mit einer effektiven Menge einer Verbindung, gemäß Anspruch 1-15 in Kontakt kommen.

17. Eine in vitro Methode um die Bildung falschgefalteter Proteine zu hemmen, wobei die spezifische APIase, welche diese Faltung katalysiert, mit einer effektiven Menge eine Verbindung gemäß Anspruch 1 - 15 inhibiert wird.

18. Die Methoden von Anspruch 16 oder 17, worin die spezifische APIase Dnak ist.

19. Verwendung einer Verbindung, gemäß Anspruch 1-15 zur Herstellung eines Arzneimittels zur Inhibierung von APIase.

20. Verwendung nach Anspruch 19, wobei die APIase Dnak ist.

21. Verwendung gemäß Anspruch 19 oder 20 zur Hemmung des Zellwachstums von hyperplastischen oder neoplastischen Krankheitsprozessen.

22. Die Methode von Anspruch 16 bis 18, wobei die Zellen eukaryotische Zellen sind.

23. Die Methode von Anspruch 16, wobei die Zellen sich aus folgender Gruppe rekrutieren: Säugetierzellen, Hefezellen, Pilzzellen.

24. Die Methode von Anspruch 17, wobei die falsche Faltung von Proteinen zu Krankheiten führt.

## Claims

1. A pharmaceutical composition comprising a peptide or peptide mimetic with the general basic structure
Y-R₂-R₃
wherein Y is a saturated or unsaturated, linear or branched chain fatty acid which is linked to the amino acid or the amino acid mimetic R₂ by a C(O)NH bond and wherein the amino acid or amino acid mimetic R₃, is linked to R₂ by a C(O)NH bond via its amino group, wherein R₂ comprises all natural amino acids or their mimetics and R₃ comprises the amino acids methionine, alanine, serine, glutamic acid, leucine, lysine, isoleucine and/or glycine or their mimetics and optionally a pharmaceutically acceptable carrier.

2. The pharmaceutical composition as claimed in claim 1, wherein the peptide mimetic R₃ consists of at least 5 and not more than 29 atoms and has to be able to form a chemical bond with R₂ which corresponds to the structure of a peptide bond, wherein the basic structure of R₃ is described as H₂N-R-Z, wherein Z represents a functional group selected from nitro, sulfoxy, phosphor, amino, carboxy, sulfhydryl, wherein R represents a linear or branched hydrocarbon chain, whose length is a maximum of 14 carbon atoms and a minimum of 1 carbon atom, wherein the distance between H₂N- -Z via R is a maximum of 4 carbon atoms and a minimum of 1 carbon atom, wherein R itself can optionally have one or up to three hydroxyl, carboxylic acid, carboxylic acid ester, amide, aldehyde, ether, iminoether, hydrazide, izidoether, thiol or sulfoxime groups.

3. The pharmaceutical composition as claimed in claim 1 or 2, wherein the fatty acid has 8 to 24 carbon atoms.

4. The pharmaceutical composition as claimed in any one of claims 1 to 3, wherein the fatty

5. The pharmaceutical composition as claimed in any one of claims 1 to 4, wherein the fatty acid is mono-hydroxylated.

6. The pharmaceutical composition as claimed in any one of claims 1 to 4, wherein the fatty acid is poly-hydroxylated.

7. The pharmaceutical composition as claimed in any one of claims 1 to 6, wherein the amino acid mimetics R₂ and R₃ are the same or different and have up to 34 carbon atoms and a linear structure which can optionally be branched, a C5 to C34 carbocyclic structure or a heterocyclic structure containing S, N and O as hetero atoms and having ring sizes of 5 to 8, wherein these structures can be completely saturated or can have mono- or poly-unsaturated carbon moieties.

8. The pharmaceutical composition as claimed in claim 7, wherein R₂ and R₃ include benzoic or non-benzoic aromatic groups which can optionally be mono- or polysubstituted with functional groups selected from nitro, sulfoxy, phosphor, amino, carboxy, sulfhydryl groups.

9. The pharmaceutical composition as claimed in claim 7 or 8, wherein R₂ and R₃ are mono- or poly-halogenated, wherein halogen is selected from fluoro, chloro, bromine or iodine.

10. The pharmaceutical composition as claimed in claims 1 to 9, wherein Y is a saturated fatty acid.

11. The pharmaceutical composition as claimed in claims 1 to 9, wherein Y is an unsaturated fatty acid.

12. The pharmaceutical composition as claimed in claim 10, wherein the saturated fatty acid is selected from: propionic acid, butyric acid, isobutyric acid, valeric acid, isovaleric acid, caproic acid, enanthic acid, caprylic acid, pelargonic acid, capric acid, undecanoic acid, lauric acid, tridecanoic acid, myristic acid, pentadecanoic acid, palmitic acid, margaric acid, stearic acid, nonadecanoic acid, isotuberculostearic acid, arachic acid, behenic acid, lignoceric acid, cerotic acid and melissic acid.

13. The pharmaceutical composition as claimed in claim 12, wherein the unsaturated fatty acid is selected from: acrylic acid, crotonic acid, palmitoleic acid, oleic acid, erucic acid, sorbic acid, elaeostearic acid, arachidonic acid, clupanodonic acid, docosahexaenoic acid, elaidic acid, linolic acid and linolenic acid.

14. The pharmaceutical composition as claimed in any one of claims 1 to 13, wherein R₂ and R₃ together represent a peptide mimetic.

15. The pharmaceutical composition as claimed in claims 1 to 14, comprising a compound with the formula: 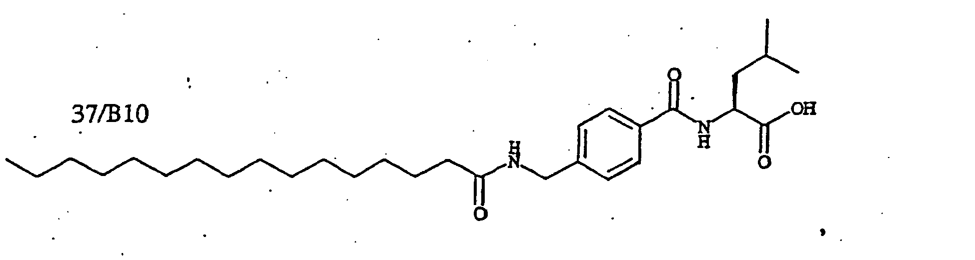 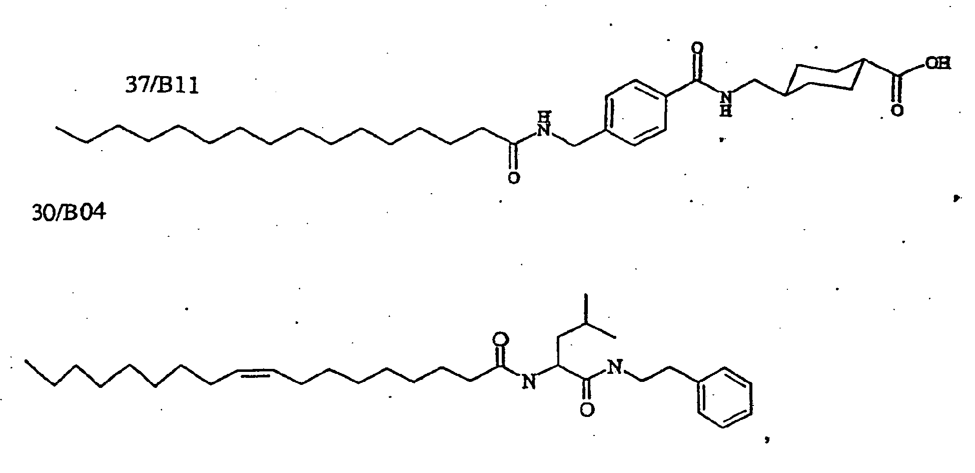 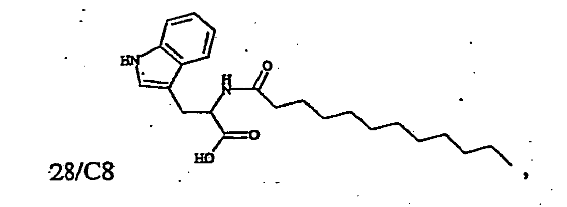 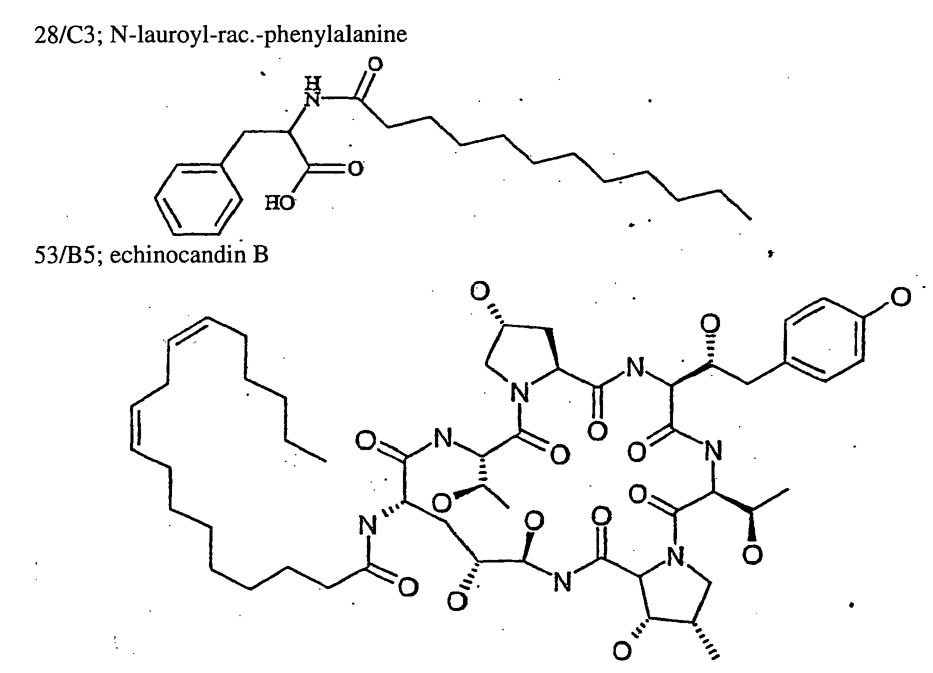 and

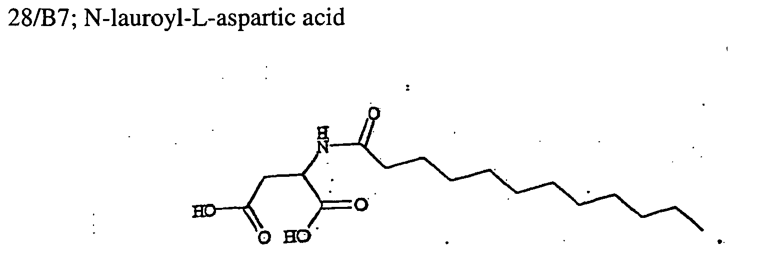

16. An in vitro method for inhibiting cell growth by inhibiting an APIase activity by contacting the corresponding cells with an effective amount of a compound according to claims 1 to 15.

17. An in vitro method for inhibiting the formation of incorrectly folded proteins, wherein the specific APIase which catalyzes this folding is inhibited with an effective amount of a compound according to claims 1 to 15.

18. The methods as claimed in claim 16 or 17, wherein the specific APIase is DnaK.

19. The use of a compound as claimed in claims 1 to 15 for the preparation of a pharmaceutical composition for inhibiting APIase.

20. The use as claimed in claim 19, wherein the APIase is DnaK.

21. The use as claimed in claim 19 or 20 for inhibiting the cell growth of hyperplastic or neoplastic disease processes.

22. The method as claimed in claims 16 to 18, wherein the cells are eukaryotic cells.

23. The method as claimed in claim 16, wherein the cells are selected from the following group: mammalian cells, yeast cells, fungal cells.

24. The method as claimed in claim 17, wherein the incorrect folding of proteins leads to diseases.

## Revendications

1. Médicament contenant un peptide ou peptidomimétique ayant la structure de base générale
Y-R₂-R₃
dans laquelle Y représente un acide gras à chaîne droite ou ramifiée, saturé ou insaturé, qui est lié à l'acide aminé ou l'acide aminé mimétique R₂ par une liaison C(O)NH et dans laquelle l'acide aminé ou l'acide aminé mimétique R₃ est lié par l'intermédiaire de son groupe amino à R₂ par une liaison C(O)NH, R₂ comprend tous les acides aminés naturels ou leurs mimétiques et R₃ les acides aminés méthionine, alanine, sérine, acide glutamique, leucine, lysine, isoleucine et/ou glycine ou leurs mimétiques, et éventuellement un support compatible du point de vue pharmaceutique.

2. Médicament selon la revendication 1, dans lequel le peptidomimétique R₃ est constitué d'au moins 5 et au maximum de 29 atomes et doit être capable de pouvoir former avec R₂ une liaison chimique telle qu'elle corresponde à la structure d'une liaison peptidique, dans lequel la structure de base de R₃ est définie par H₂N-R-Z, où Z représente un groupe fonctionnel choisi parmi nitro, sulfoxy, phospho, amino, carboxy, sulfhydryle, où R représente une chaîne droite ou ramifiée d'hydrocarbure, dont la longueur maximale contient 14 atomes de carbone et au minimum 1 atome de carbone, où la distance entre H₂N- -Z par l'intermédiaire R comporte au maximum 4 atomes de carbone et au minimum 1 atome de carbone, où R même peut être muni le cas échéant d'un ou de jusqu'à trois groupes hydroxyle, acide carboxylique, ester d'acide carboxylique, amide, aldéhyde, éther, iminoéther, hydrazide, izidoéther, thiol ou sulfoxime.

3. Médicament selon la revendication 1 ou 2, dans lequel l'acide gras présente de 8 à 24 atomes de carbone.

4. Médicament selon l'une des revendications 1 à 3, dans lequel l'acide gras présente de 14 à 16 atomes de carbone.

5. Médicament selon l'une des revendications 1 à 4, dans lequel l'acide gras est hydroxylé une fois.

6. Médicament selon l'une des revendications 1 à 4, dans lequel l'acide gras est hydroxylé plusieurs fois.

7. Médicament selon l'une des revendications 1 à 6, dans lequel les acides aminés mimétiques R₂ ou R₃ sont identiques ou différents et ont jusqu'à 34 atomes de carbone et ont une structure linéaire qui peut être éventuellement ramifiée, une structure carbocyclique de C5 à C34 ou une structure hétérocyclique, contenant S, N et O comme hétéroatomes et des grandeurs de cycle de 5 à 8, ces structures pouvant être complètement saturées ou disposer de groupements de carbone saturés une fois ou plusieurs fois.

8. Médicament selon la revendication 7, dans lequel R₂ et R₃ comprennent des aromates benzoïdes ou non benzoïdes, qui peuvent être substitués éventuellement une fois ou plusieurs fois par des groupes fonctionnels choisis par mi les groupes nitro, sulfoxy, phospho, amino, carboxy, sulfhydryle.

9. Médicament selon la revendication 7 ou 8, dans lequel R₂ et R₃ sont halogénés une ou plusieurs fois, l'halogène étant choisi parmi le fluor, le chlore, le brome ou l'iode.

10. Médicament selon les revendications 1 à 9, dans lequel Y est un acide gras saturé.

11. Médicament selon les revendications 1 à 9, dans lequel Y est un acide gras insaturé.

12. Médicament selon la revendication 10, dans lequel l'acide gras saturé est choisi parmi : l'acide propionique, l'acide butyrique, l'acide isobutyrique, l'acide valérianique, l'acide isovalérianique, l'acide caproïque, l'acide oenanthique, l'acide caprylique, l'acide pelargonique, l'acide caprique, l'acide undécanique, l'acide laurique, l'acide tridécanique, l'acide myristique, l'acide pentadécanique, l'acide palmitique, l'acide margarique, l'acide stéarique, l'acide nonadécanique, l'acide isoturbercolostéarique, l'acide arachique, l'acide béhénique, l'acide lignocérique, l'acide cérotique et l'acide milissique.

13. Médicament selon la revendication 12, dans lequel l'acide gras insaturé est choisi parmi : l'acide acrylique, l'acide crotonique, l'acide palmitoléique, l'acide oléique, l'acide érucique, l'acide sorbique, l'acide élæostéarique, l'acide arachidonique, l'acide clupanodonique, l'acide docosahexaénique, l'acide élaïdique, l'acide linoléique et l'acide linolénique.

14. Médicament selon l'une des revendications 1 à 13, dans lequel R₂ et R₃ représentent ensemble un peptidomimétique.

15. Médicament selon les revendications 1 à 14, comprenant un composé ayant la formule 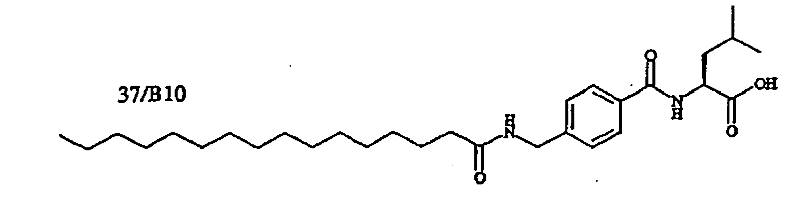 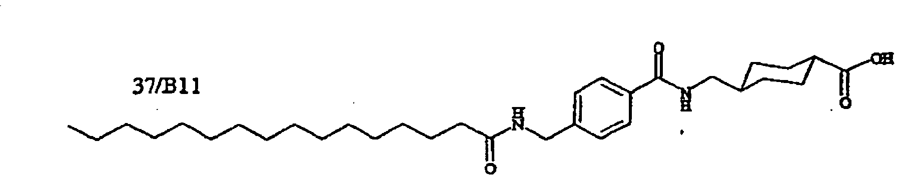 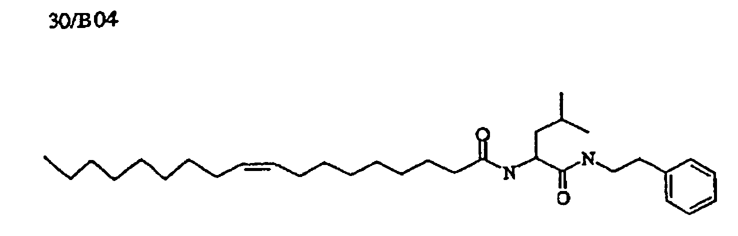 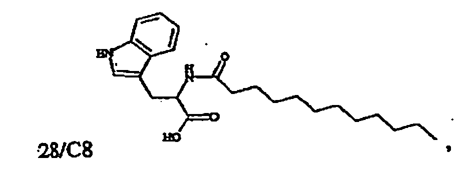 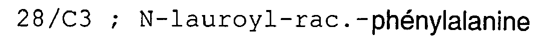 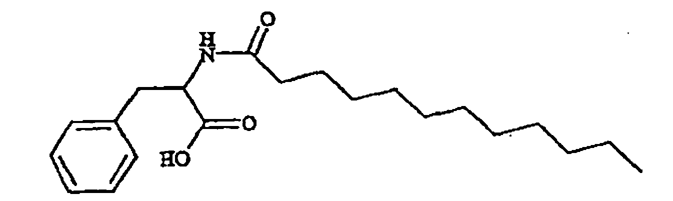 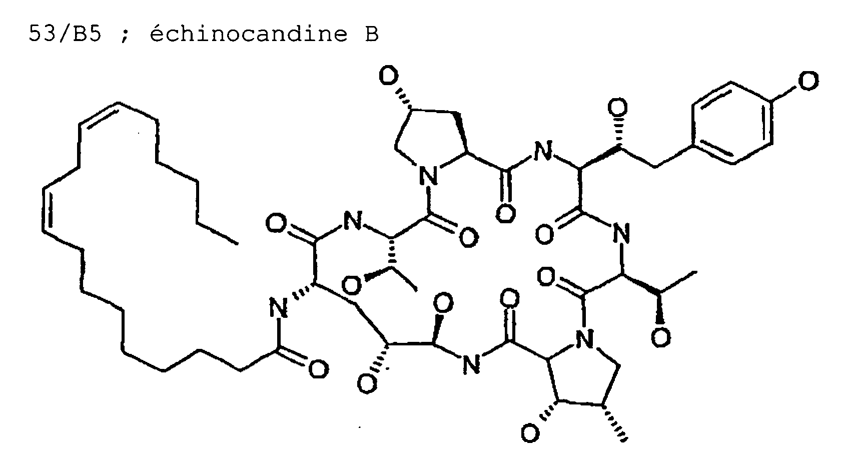 et

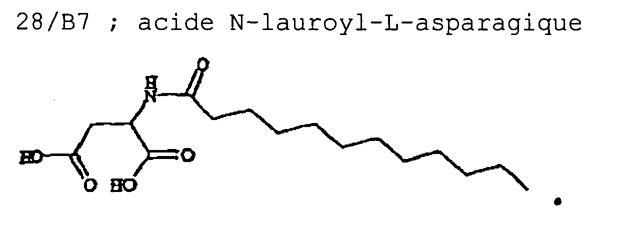

16. Procédé in vitro pour inhiber la croissance cellulaire par inhibition d'une activité APIase, dans lequel les cellules concernées viennent en contact avec une quantité effective d'un composé selon les revendications 1 - 15.

17. Procédé in vitro pour bloquer la formation de protéines mal pliées, dans lequel la APIase spécifique qui catalyse ce pliage est inhibée avec une quantité effective d'un composé selon les revendications 1 - 15.

18. Procédés selon la revendication 16 ou 17, dans lesquels la APIase spécifique est DnaK.

19. Utilisation d'un composé selon les revendications 1 - 15 pour la fabrication d'un médicament pour l'inhibition de la APIase.

20. Utilisation selon la revendication 19, dans laquelle la APIase est **DnaK**.

21. Utilisation selon la revendication 19 ou 20 pour le blocage de la croissance cellulaire de processus pathologiques hyperplasiques ou néoplasiques.

22. Procédé selon les revendications 16 à 18, dans lequel les cellules sont des cellules eukaryotiques.

23. Procédé selon la revendication 16, dans lequel les cellules sont choisies dans le groupe suivant : cellules de mammifères, cellules de levures, cellules de champignons.

24. Procédé selon la revendication 17, dans lequel le pliage défectueux de protéines conduit à des maladies.
